# EUROPEAN PATENT SPECIFICATION

(11) **EP 1 791 967 B1**
(45) Date of publication and mention of the grant of the patent: **27.11.2013**
(21) Application number: 05810433.2
(22) Date of filing: 21.09.2005
(51) Int. Cl.: C12P 21/04, G01N 33/53, A61K 39/395

(54) **METHODS AND COMPOSITIONS RELATING TO MANNURONIC ACID SPECIFIC BINDING PEPTIDES**
VERFAHREN UND ZUSAMMENSETZUNGEN IN VERBINDUNG MIT MANNURONSÄURESPEZIFISCHEN BINDUNGSPEPTIDEN
PROCEDES ET COMPOSITIONS SE RAPPORTANT A DES PEPTIDES SE LIANT SPECIFIQUEMENT A L'ACIDE MANNURONIQUE

(30) Priority: 21.09.2004 US 612083 P
(43) Date of publication of application: 06.06.2007
(73) Proprietor: THE BRIGHAM AND WOMEN'S HOSPITAL, INC., Boston, MA 02115 (US)
(72) Inventor: PIER, Gerald, B., Brookline, MA 02446 (US)
(74) Representative: Lahrtz, Fritz
(86) International application number: PCT/US2005/033962
(87) International publication number: WO 2006/034395

(56) References cited:
- EP-A- 1 243 256
- WO-A-94/08617
- WO-A-02/094854
- CN-A- 1 401 786
- US-A- 5 169 840
- US-A- 6 121 441
- VERMEER H J ET AL: "Immunodiagnostically applicable monoclonal antibodies to the circulating anodic antigen of Schistosoma mansoni bind to small, defined oligosaccharide epitopes." PARASITOLOGY RESEARCH JUL 2003, vol. 90, no. 4, July 2003 (2003-07), pages 330-336, XP002513961 ISSN: 0932-0113
- JOHNSON MARGARET A ET AL: "Saturation transfer difference 1D-TOCSY experiments to map the topography of oligosaccharides recognized by a monoclonal antibody directed against the cell-wall polysaccharide of group A streptococcus." JOURNAL OF THE AMERICAN CHEMICAL SOCIETY 25 DEC 2002, vol. 124, no. 51, 25 December 2002 (2002-12-25), pages 15368-15374, XP002513962 ISSN: 0002-7863
- PEDERSEN S.S. ET AL.: 'Immunoglobulin A and Immunoglobulin G antibody responses to alginates from Pseudomonas aeruginosa in patients with cystic fibrosis' JOURNAL OF CLINICAL MICROBIOLOGY vol. 28, April 1998, pages 747 - 755, XP003003704
- THU B. ET AL.: 'Alginate polycation microcapsules' BIOMATERIALS vol. 17, 1996, pages 1031 - 1040, XP004032690
- EPPS D.E. ET AL.: 'A fluorescence resonance energy transfer method for measuring the binding of inhibitors to stromelysin' ANALYTICAL BIOCHEMISTRY vol. 275, 1999, pages 141 - 147, XP003003705

### SEQUENCE LISTING

<110> The Brigham and Women's Hospital, Inc.
<120> METHODS AND COMPOSITIONS RELATING TO MANNURONIC ACID SPECIFIC
   BINDING PEPTIDES
<130> B0801.70307WO00
<140> Not yet assigned
   <141> 2005-09-21
<150> US 60/612,083
   <151> 2004-09-21
<160> 15
<170> PatentIn version 3.3
<210> 1
   <211> 18
   <212> DNA
   <213> Artificial sequence
<220>
   <223> Synthetic oligonucleotide
<400> 1
   ccatggactg gacctgga 18
<210> 2
   <211> 20
   <212> DNA
   <213> Artificial sequence
<220>
   <223> Synthetic oligonucleotide
<400> 2
   atggacatac tttgttccac 20
<210> 3
   <211> 20
   <212> DNA
   <213> Artificial sequence
<220>
   <223> Synthetic oligonucleotide
<400> 3
   ccatggagyy kgggctgagc 20
<210> 4
   <211> 19
   <212> DNA
   <213> Artificial sequence
<220>
   <223> Synthetic oligonucleotide
<400> 4
   atgaacayct gtggttctt 19
<210> 5
   <211> 20
   <212> DNA
   <213> Artificial sequence
<220>
   <223> Synthetic oligonucleotide
<400> 5
   atggggtcaa ccgccatcct 20
<210> 6
   <211> 20
   <212> DNA
   <213> Artificial sequence
<220>
   <223> Synthetic oligonucleotide
<400> 6
   atgtctgtct ccttcctcat 20
<210> 7
   <211> 17
   <212> DNA
   <213> Artificial sequence
<220>
   <223> Synthetic oligonucleotide
<400> 7
   cagcgggaag accttgg 17
<210> 8
   <211> 33
   <212> DNA
   <213> Artificial sequence
<220>
   <223> Synthetic oligonucleotide
<400> 8
   agatctctca ccatggccrg cttccctctc ctc 33
<210> 9
   <211> 33
   <212> DNA
   <213> Artificial sequence
<220>
   <223> Synthetic oligonucleotide
<400> 9
   agatctctca ccatgacctg cttccctctc ctc 33
<210> 10
   <211> 29
   <212> DNA
   <213> Artificial sequence
<220>
   <223> Synthetic oligonucleotide
<400> 10
   agatctctca ccatggcctg ggctctgct 29
<210> 11
   <211> 33
   <212> DNA
   <213> Artificial sequence
<220>
   <223> Synthetic oligonucleotide
<400> 11
   agatctctca ccatgacttg gatccctctc ttc 33
<210> 12
   <211> 33
   <212> DNA
   <213> Artificial sequence
<220>
   <223> Synthetic oligonucleotide
<400> 12
   agatctctca ccatggcatg gatccctctc ttc 33
<210> 13
   <211> 33
   <212> DNA
   <213> Artificial sequence
<220>
   <223> Synthetic oligonucleotide
<400> 13
   agatctctca ccatggcctg gacccctctc tgg 33
<210> 14
   <211> 33
   <212> DNA
   <213> Artificial sequence
<220>
   <223> Synthetic oligonucleotide
<400> 14
   agatctctca ccatggcctg gatgatgctt ctc 33
<210> 15
   <211> 21
   <212> DNA
   <213> Artificial sequence
<220>
   <223> Synthetic oligonucleotide
<400> 15
   ggagggcggg aacagagtga c 21

## Description

### Related Applications

This application claims priority under 35 U.S.C. §119(e) from U.S. provisional application serial number 60/612,083, filed September 21, 2004, the entire contents of which is incorporated by reference herein.

### Government Support

This invention was made in part with government support under grant number RO 1 A148917 from The National Institutes of Health, National Institute of Allergy and Infectious Diseases. The Government may retain certain rights in the invention.

### Field of the Invention

The invention relates to epitopes on alginate from bacteria and other sources, and methods and compositions related thereto.

### Background of the Invention

The over-production of the cell surface polysaccharide alginate (also called mucoid exopolysaccharide or MEP) leading to phenotypically mucoid colonies of *Pseudomonas aeruginosa* is closely associated with isolates from the respiratory tract of cystic fibrosis (CF) patients. Mucoid strains are also obtained from patients with chronic *P. aeruginosa* urinary tract infections (1). Clinical non-mucoid isolates are primarily obtained from the blood, respiratory or urinary tracts of immunocompromised hosts and express low-levels of the alginate antigen. Mucoid *P. aeruginosa* will infect over 80% of CF patients (2). *P. aeruginosa* infection in CF is initiated early in life (3, 4) but respiratory decline is clearly associated with the emergence of the alginate over-producing mucoid strains (5, 6). In spite of a potent host immune response, the organism persists for years, is never cleared, and ultimately is responsible for the majority of the morbidity and early mortality seen in CF.

A small percentage of older (>12 years old) CF patients escape chronic infection with *P. aeruginosa* by acquiring phagocyte-dependent killing antibody specific to the alginate antigen (7, 8). Although non-killing and non-protective alginate-specific antibodies do develop in CF patients (7) and these antibodies activate complement, there is little deposition of the C3 opsonins C3b and iC3b onto the alginate on the outer cell surface (9). In contrast, the alginate-specific antibodies that mediate killing deposit high levels of these complement-derived opsonins (9) which are absolutely required for efficient phagocytosis and killing of mucoid *P. aeruginosa* (10).

In spite of numerous attempts at active immunization of humans with alginate or alginate conjugate vaccines (11-13) it has been difficult to consistently elicit phagocyte-dependent killing and protective antibodies in the majority of vaccinates including healthy human plasma donors (14). This may reflect an essential property of the alginate antigen, wherein any protective epitopes are poorly immunogenic, allowing chronic infection to persist in the otherwise immunocompetent CF host.

Many of the murine monoclonal and polyclonal antibodies to alginate that have been generated to date that mediate killing of mucoid strains of *P. aeruginosa* and protect animals against infection are specific to epitopes formed by acetylation of the C2 and C3 hydroxyl groups of the mannuronic acid constituents of alginate (15, 16).

### Summary of the Invention

The invention is based in part on the discovery of a protective epitope of alginate. The epitope was identified through characterization of recently identified fully human monoclonal antibodies to alginate that not only mediate killing and protect against infection with highly mucoid CF isolates ofP. *aeruginosa,* but also can kill and protect against non-mucoid, low-alginate producing strains (17,18) in a murine model of acute pneumonia (19). It was discovered according to the invention that such protective human monoclonal antibodies are directed against epitopes that comprise the carboxylic-acid components of the alginate molecule, and more specifically the carboxylic acid (or carboxylate group) on carbon 6 (C6) of the mannuronic acid constituent of alginate. This antigenic specificity is distinct from that previously described for monoclonal antibodies and polyclonal antisera that have killing and protective activity for mucoid (15), but not non-mucoid, strains (20).

Thus, based on this discovery, the invention provides compositions comprising binding partners of the alginate epitope, including binding peptides such as antibodies or fragment thereof. The invention further provides methods of using the alginate epitope and/or its binding partner. Such methods include screening methods to identify further binding partners of the alginate epitope, as well as methods for inducing active and passive immunity in subjects that would benefit prophylactically or therapeutically from such treatment.

In one aspect, the invention provides a method for identifying a mannuronic acid binding peptide comprising contacting a candidate peptide to isolated beta-1,4 linked homopolymer of D-mannuronic acid, and determining whether the candidate peptide binds to an epitope that comprises an intact carboxyl group on C6 of the isolated beta-1,4 linked homopolymer of D-mannuronic acid.

In one embodiment, the method further comprises determining whether the candidate peptide is a mannuronic acid binding peptide based on comparison with a control. In one embodiment, the control is a negative control level of binding and the candidate peptide is a mannuronic acid binding peptide if the test level of binding is greater than the negative control level of binding. In a related embodiment, the test level of binding is greater than the negative control level of binding by at least 2-fold, at least 3-fold, at least 4-fold, at least 5-fold, at least 10-fold, at least 20-fold, at least 50-fold or at least 100-fold. In another embodiment, the control is a positive control level of binding and the candidate peptide is a mannuronic acid binding peptide if the test level of binding is substantially equal to or greater than the positive control level of binding. In a related embodiment, the test level of binding is at least 50%, at least 75%, or at least 90% of the positive control level of binding.

In one embodiment, the beta-1,4 linked homopolymer of D-mannuronic acid and candidate peptide are both soluble. In another embodiment, the beta-1,4 linked homopolymer of D-mannuronic acid and the candidate peptide are both labeled. In a related embodiment, the beta-1,4 linked homopolymer of D-mannuronic acid and the candidate peptide are labeled with a FRET pair.

In another embodiment, the method further comprises excluding candidate peptides that bind to mannoside and/or excluding candidate peptides that bind to guluronic acid.

It is to be understood that various embodiments recited above and below are equally applicable to the various aspects of the invention. For the sake of brevity such embodiments will not be reiterated, however, it is to be understood that they apply equally to various aspects of the invention, unless explicitly stated otherwise.

Thus, in another aspect, the invention provides another method for identifying a mannuronic acid binding peptide comprising determining a level of binding of monoclonal antibody F428 or F429 (or an antigen-binding fragment thereof) to isolated beta-1,4 linked homopolymer of D-mannuronic acid in the presence and absence of a candidate peptide, wherein a candidate peptide that reduces the level of binding of monoclonal antibody F428 or F429 or an antigen-binding fragment thereof to isolated beta-1,4 linked homopolymer of D-mannuronic acid, and does not bind to the monoclonal antibody, or antigen-binding fragment thereof is a mannuronic acid binding peptide.

According to the method, a candidate peptide that reduces the level of binding of monoclonal antibody F428 or F429 (or an antigen-binding fragment thereof) to a beta-1,4 linked homopolymer of D-mannuronic acid, and does not bind to the monoclonal antibody (or antigen-binding fragment thereof) is a mannuronic acid binding peptide.

In one embodiment, the candidate peptide reduces the level of binding of monoclonal antibody F428 or F429 or an antigen-binding fragment thereof to isolated beta-1,4 linked homopolymer of D-mannuronic acid by at least 2-fold, at least 3-fold, at least 4-fold, at least 5-fold, at least 10-fold, at least 20-fold, at least 50-fold or at least 100-fold.

In one embodiment the monoclonal antibody is F428 or an antigen-binding fragment thereof, while in another embodiment the monoclonal antibody is F429 or an antigen-binding fragment thereof.

In one embodiment, the candidate peptide is an antibody or a fragment thereof. The antibody may be a single chain antibody, but it is not so limited.

The candidate peptide may be a library member such as a synthetic library member or a naturally occurring library member.

In one embodiment, the beta-1,4 linked homopolymer of D-mannuronic acid has a length of two monomers, four monomers, six monomers, ten monomers, fifteen monomers or twenty monomers. In another embodiment, the beta-1,4 linked homopolymer of D-mannuronic acid has a length of at least 21, at least 50, at least 100, at least 500 monomers, at least 1,000 monomers, at least 5,000 monomers, at least 10,000 monomers, or at least 100,000 monomers.

In one embodiment, the beta-1,4 linked homopolymer of D-mannuronic acid is acetylated. For example, the beta-1,4 linked homopolymer of D-mannuronic acid may be O-acetylated at one or both C2 or C3. In another embodiment, the beta-1,4 linked homopolymer of D-mannuronic acid is not acetylated.

The screening methods may further comprise prescreening or postscreening steps such as determining if the candidate peptide binds to mannoside and/or guluronic acid Candidate peptides that do so are excluded.

In one embodiment, the monoclonal antibody or antigen-binding fragment thereof, the beta-1,4 linked homopolymer of D-mannuronic acid and the candidate peptide are soluble. In various embodiments, the monoclonal antibody or antigen-binding fragment thereof is fixed, or the beta-1,4 linked homopolymer of D-mannuronic acid is fixed, or the candidate peptide is fixed. In various other embodiments, the monoclonal antibody or antigen-binding fragment thereof is labeled, or the beta-1,4 linked homopolymer of D-mannuronic acid is labeled, or the candidate peptide is labeled, or some combination thereof is labeled. For example, the monoclonal antibody or antigen-binding fragment thereof and the beta-1,4 linked homopolymer of D-mannuronic acid may both be labeled. As another example, the monoclonal antibody or antigen-binding fragment thereof and the beta-1,4 linked homopolymer of D-mannuronic acid may be labeled with a FRET pair.

The test level of binding may be measured by any detection system known including but not limited to a charge coupled device (CCD) detection system, an electron spin resonance (ESR) detection system, an electrical detection system, an electron microscopy detection system, a confocal laser microscopy detection system, a photographic film detection system, a fluorescent detection system, a chemiluminescent detection system, an enzyme detection system, an atomic force microscopy (AFM) detection system, a scanning tunneling microscopy (STM) detection system, a scanning electron microscopy detection system, an optical detection system, an electron density detection system, a refractive index system, a nuclear magnetic resonance (NMR) detection system, a near field detection system, a total internal reflection (TIR) detection system, or a electromagnetic detection system.

The beta-1,4 linked homopolymer of D-mannuronic acid may be conjugated to at least one guluronic acid monomer.

The beta-1,4 linked homopolymer of D-mannuronic acid may be derived from bacteria such as but not limited to an *Pseudomonas* spp. (e.g., *P. aeruginosa, P. syringae, P. fluorescens, P. putida* or *P. mendocina*) or an *Azotobacter* spp. (e.g., *A. vinelandii*)*.* The beta-1,4 linked homopolymer of D-mannuronic acid may be derived from a plant such as but not limited to seaweed. Alternatively, the beta-1,4 linked homopolymer of D-mannuronic acid is synthesized de novo.

In one embodiment, the candidate peptide is not positively charged.

The invention describes other forms of screening methods as will be appreciated in view of the invention. These include a method for identifying a mannuronic acid specific antibody or fragment thereof comprising contacting a candidate antibody or fragment thereof to a beta-1,4 linked homopolymer of D-mannuronic acid, and determining whether the candidate antibody or fragment binds to the beta-1,4 linked homopolymer of D-mannuronic acid, wherein the candidate antibody or fragment thereof was raised against alginate.

Another screening method for identifying a mannuronic acid specific antibody or fragment thereof comprises contacting a candidate antibody or fragment thereof to alginate, and determining whether the candidate antibody or fragment binds to alginate, wherein the candidate antibody or fragment thereof was raised against a beta-1,4 linked homopolymer of D-mannuronic acid. The method may further comprise contacting the candidate antibody or fragment thereof to guluronic acid and excluding the candidate antibody or fragment thereof if it binds to guluronic acid.

Yet another method for identifying a mannuronic acid specific antibody or fragment thereof comprises selecting a candidate antibody or fragment thereof that binds to alginate and excluding a candidate antibody or fragment thereof that binds to guluronic acid, wherein the candidate antibody or fragment thereof was raised against alginate.

In other aspects, the invention provides compositions and describes kits.

In one aspect, the invention describes a pharmaceutical preparation comprising an effective amount of a beta-1,4-linked D-mannuronic acid having a length ranging from four to less than 1000 monomeric units, and a pharmaceutically-acceptable carrier. The preparation further comprises an adjuvant. The beta-1,4-linked D-mannuronic acid may be derived from sources as listed above, including mucoid strains of bacteria such as those recited above, or synthesized de novo. The beta-1,4-linked D-mannuronic acid may or may not be acetylated, as described above. The beta-1,4-linked D-mannuronic acid may be 4-6 monomers in length. It may also be less than 10 monomers in length, less than 20 monomers in length, less than 50 monomers in length, less than 100 monomers in length, less than 150 monomers in length, less than 200 monomers in length, less than 250 monomers in length, less than 500 monomers in length, or less than 750 monomers in length. In one embodiment, the homopolymer is isolated.

In one embodiment, the peptide is a monoclonal antibody such as but not limited to a human or humanized antibody. The peptide may also be an antibody fragment or a single chain antibody. In one embodiment, the peptide is isolated.

Another aspect of the invention provides a composition comprising a peptide that binds specifically to an epitope comprising a carboxyl group on C6 of a mannuronic acid, wherein the peptide is none of monoclonal antibody F428, F429, or a monoclonal antibody comprising the amino acid sequences of all complementarity determining regions of F428 or F429.

The composition may be a pharmaceutical preparation, thus optionally further comprising a pharmaceutically-acceptable carrier.

In still other aspects, the invention describes kits comprising any of the aforementioned compositions and preparation. Preferably, the kits comprise containers to house or hold the composition or preparations, and optionally further comprise instructions, other reagents and utensils and/or an outer housing. The compositions may be attached to a surface of a solid substrate provided in the kit The solid substrate may be a dipstick, a slide, a plastic dish, a petri plate or well, and the like. The compositions may be labeled.

In yet other aspects, the invention describes treatment methods employing the compositions and preparations of the invention.

In one aspect, the invention describes a method for inducing an anti-mannuronic acid specific immune response in a non-rodent subject comprising administering a non-rodent subject in need thereof a beta-1,4-linked D-mannuronic acid in an effective amount to induce an immune response. The method may further comprise administering an adjuvant to the subject. The immune response may be specific to a bacterial infection, such as a *Pseudomonas* infection. The beta-1,4-linked D-mannuronic acid can be derived from various sources or synthesized de novo as described above. It can be acetylated or not acetylated as described above. The beta-1,4-linked D-mannuronic acid may be 4-6 monomers in length. It may also be less than 10 monomers in length, less than 20 monomers in length, less than 50 monomers in length, less than 100 monomers in length, less than 150 monomers in length, less than 200 monomers in length, less than 250 monomers in length, less than 500 monomers in length, less than 750 monomers in length, is less than 1000 monomers in length, less than 10,000 monomers in length, or less than 100,000 monomers in length. The beta-1,4-linked D-mannuronic acid may be isolated.

In another aspect, the invention provides a method for inducing passive immunity in a subject comprising administering to a subject in need thereof an antibody or fragment thereof that binds to an epitope that comprises an intact carboxyl group on C6 of a mannuronic acid in an effective amount to induce passive immunity, wherein the antibody or antibody fragment is not monoclonal antibody F428 or F429 or an antigen-binding fragment thereof, and is not a polyclonal antibody. The antibody or fragment thereof may be isolated.

In one embodiment, the passive immunity is against bacteria such as a *Pseudomonas* spp. (e.g., *Pseudomonas* spp. is *P. aeruginosa, P. fluorescens. P. syringae*, *P. putida* or *P. mendocina*) or an *Azotobacter* spp. (*A. vinelandii*)*.* The bacteria may be of a mucoid or non-mucoid strain.

The antibody or fragment thereof may be a monoclonal antibody such as a human or humanized antibody, an antibody fragment, or a single chain antibody.

In one embodiment, the subject has or is at risk of a *Pseudomonas* infection such as *P. aeruginosa* infection, *P. syringae* infection, *P. fluorescens* infection, *P. putida* infection or *P. mendocina* infection, In another embodiment, the subject has or is at risk of an *Azotobacter* infection such as *A*. *vinelandit* infection,

In other embodiments, the subject has a respiratory condition, or the subject is receiving mechanical ventilation, or the subject has ventilation-associated pneumonia, or the subject has cystic fibrosis, or the subject has cancer, or the subject has been burned. Preferably, the subject is a human.

These aspects of the invention, as well as various advantages and utilities, will be more apparent with reference to the detailed description of the invention.

### Brief Description of the Drawings

FIG. 1 is a diagram of the structure of alginate.
FIG. 2 is a diagram showing epitopes of alginate.
FIG. 3A shows the binding properties of mAb F429γ1 to alginate from 8 different mucoid strains (numbered with symbol for binding curve) of *P*. *aeruginosa.*
FIG. 3B shows the binding properties ofmAb F429γ1 and control IgG1 myeloma to alginate from *P. aeruginosa* strain 2192, polymannuronic acid and polymannuronic acid acetylated on the hydroxyl groups.
FIG. 3C shows the binding properties of mAb F429γ1 to intact and carbodiimide reduced *P. aeruginosa* alginate or polymannuronic acid.
FIG. 3D shows the comparative binding of 4 human mAbs to intact and reduced polymannuronic acid (indicated by form of antigen).
FIG. 4A shows the phagocyte-dependent killing activity ofmAb F429γ1 against *P*.
   *aeruginosa* strains at a mAb concentration of 4-25 µg/ml of 4 mucoid clinical isolates from CF patients, 5 non-mucoid clinical isolates from CF patients, and two laboratory strains,
   PA14 and PAO1.
FIG. 4B shows the phagocyte-dependent killing activity ofmAb F429γ1 against *P*.
   *aeruginosa* strains at the indicated mAb concentration of 4 isolates of *P*. *aeruginosa* from the blood.
FIG. 4C shows the phagocyte-dependent killing of mucoid strain 324 by mAb F429 in the context of 4 different human IgG isotypes. Bars represent means of quadruplicate determinations and error bars, where shown, represent SEM. When error bars are not shown SEM was <5%. Killing of >40% was statistically significant at P<.05. Biologically significant protection as defined by a correlation with protective immunity is generally seen in sera manifesting killing of >60%.
FIG. 5A is a phase-contrast micrograph representative of images from lungs taken immediately after infection showing elaboration of alginate in vivo by *P. aeruginosa.* Lung tissues from mice infected intranasally (IN) with *P. aeruginosa* strain PAO1, which does not elaborate alginate in vitro, were stained for production of alginate as visualized by immunofluorescence. No fluorescence indicative of alginate elaboration was seen. Magnification X 400.
FIG. 5B shows detection of alginate expression in vivo by *P. aeruginosa* PAO1 1 hour post infection using mAb F429γ1. Magnification X 400.
FIG. 5C shows detection of alginate expression in vivo by *P. aeruginosa* PAO1 1 hour post infection using polyclonal antibodies to an alginate-KLH conjugate. Magnification X 400.
FIG. 5D shows detection of alginate expression in vivo by *P. aeruginosa* PAO1 1 hour post infection using polyclonal antibodies to an alginate-KLH conjugate. Magnification X 1000.
FIG. 6A shows promotion of clearance of mucoid *P. aeruginosa* strains from lungs of infected mice by mAb F429γ1 and clearance of strain FRD1 at 2, 4 and 18 hours post-infection. Numbers with percent (%) sign in open bars representing results with mAb F429γ1 indicate percent reduction in CFU compared with control IgG1. P values determined by unpaired, two-sided t-test.
FIG. 6B shows promotion of clearance of mucoid *P. aeruginosa* strains from lungs of infected mice by mAb F429γ1 and clearance of strains 8050 and 2192 at 4 h post infection. Numbers with percent (%) sign in open bars representing results with mAb F429γ1 indicate percent reduction in CFU compared with control IgG1. P values determined by unpaired, two-sided t-test.

It is to be understood that the drawings are not required for enablement of the invention.

### Detailed Descriptiton of the Invention

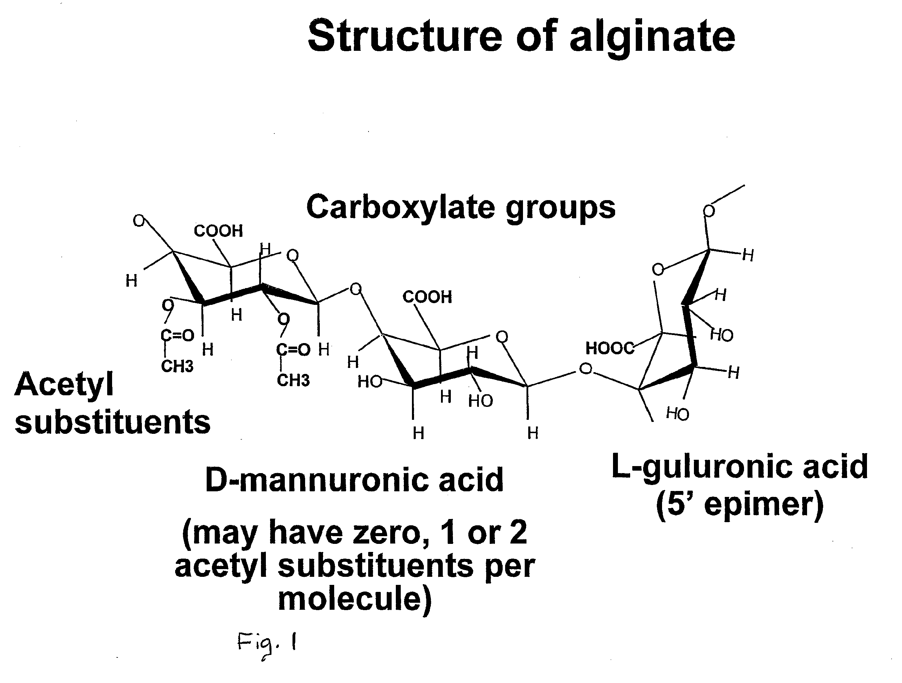

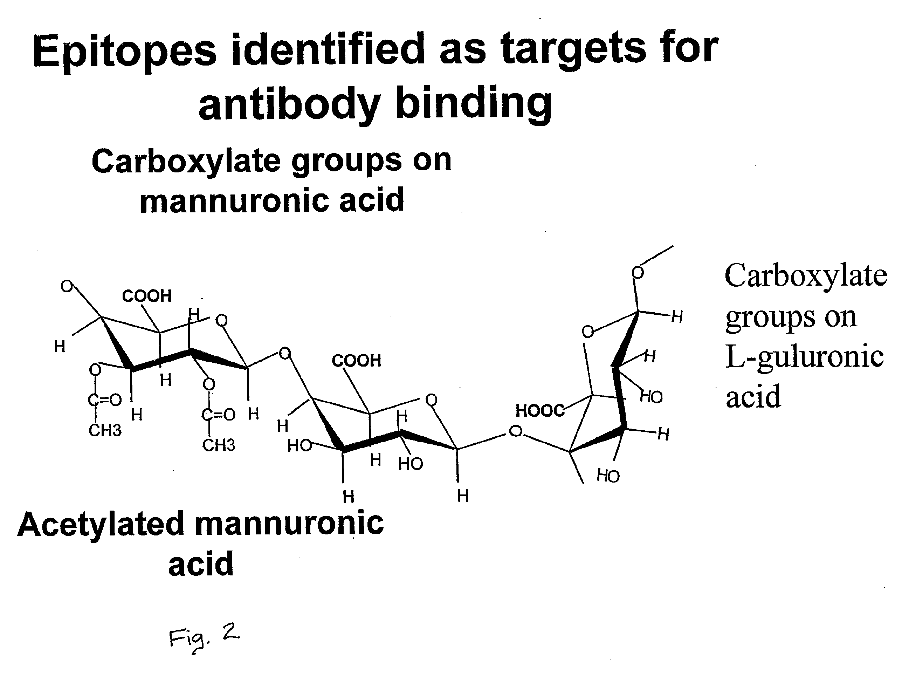

The invention is based in part on the identification of an epitope recognized by a number of opsonophagocytic antibodies specific for alginate (also referred to as mucoid exopolysaccharide or MEP). According to the invention, the epitope was found to be the carboxylic acid (or carboxylate group) on the sixth carbon (C6) of mannuronic acid monomers present in alginate. Alginate is a random co-polymer of beta-1,4 linked D-mannuronic acid and L-guluronic acid. The structure of a portion of alginate is shown in FIG. 1. The Figure shows two mannuronic acid monomers, one of which is acetylated and the other of which is not. The Figure also shows the 5' epimer L-guluronic acid which is not acetylated (at least in the Figure) but which contains a carboxylic acid (or carboxylate group) at the C6 position as well. Native alginate (i.e., naturally occurring alginate) has a random structure such that virtually any sequence of mannuronic acid and guluronic acid is possible except for a mannuronic acid-guluronic acid alternating repeat for the entire length of the molecule. Monoclonal antibodies to the guluronic acid moiety of alginate have been reported previously. These antibodies do not recognize the carboxylate group on C6 of mannuronic acid. FIG. 1 further demonstrates that D-mannuronic acid may be acetylated at 0, 1 or 2 positions per monomer. Mannuronic acid may be O-acetylated at one or both of the C2 and C3 positions, as shown in FIGS. 1 and 2. Binding of mAbs F428γ1 and F429γ1 to alginate is not dependent on acetylation, as these mAbs have been shown to bind to acetylated as well as deacetylated alginate with equal affinity. FIG. 2 shows the various epitopes that have been identified and/or contemplated in alginate, either in accordance with the invention or previously known.

The invention exploits the finding of the carboxylated C6 of mannuronic acid by providing methods for identifying compounds that will bind to alginate at this particular epitope (referred to herein as "binding partners", of which "binding peptides" are an example). The invention further provides a composition of compounds that comprise the epitope and/or the afore-mentioned binding partners. Also provided is a method for inducing passive immunity. These and other aspects of the invention will be discussed in greater detail herein.

The epitope discovered according to the invention is the carboxylic acid (or carboxylate group) on C6 of the mannuronic acid moiety of alginate. Thus, at a minimum, immunogenic compounds used in the screening and treatment methods of the invention comprise this carboxylate group on C6, or otherwise have an identical structure. Examples of such an immunogenic compound include a mannuronic acid monomer, oligomer (i.e., 2-20 monomers in length) and polymer (i.e., 21 or more monomers in length). For the sake of convenience and brevity, various aspects and embodiments refer to mannuronic acid as an exemplary immunogenic compound according to the invention. However it is to be understood that unless otherwise explicitly stated, any reference to mannuronic acid is equally applicable to the carboxylic acid (or carboxylate group) on C6 of mannuronic acid. Thus, the recitation of mannuronic acid in various aspects and embodiments therefore is not to be construed as limiting the invention.

As used herein, "an epitope containing fragment of mannuronic acid" refers to a fragment of mannuronic acid that contains the C6 carboxylic acid moiety discovered according to the invention. This term does not refer to other epitopes previously identified on mannuronic acid (see, for example, FIG. 2 for other epitopes).

As used herein, the term "mannuronic acid" encompasses mannuronic acid monomers, oligomers and polymers, unless otherwise specified. As used herein, a mannuronic acid "oligomer" has a length of 2 to 20 monomers (i.e., it has a length or2, 3,4,5,6, 7, 8, 9, 10, 11, 12,13, 14, 15, 16, 17,18,19 or 20 monomers). As used herein, a mannuronic acid "polymer" has a length of at least 21 monomers to in excess of 100,000 monomers, in some instances. Its length may be at least 25, at least 50, at least 75, at least 100, at least 200, at least 300, at least 400, at least 500, at least 750, at least 1000, at least 5000, at least 10,000, at least 25,000, at least 50,000, at least 75,000, or at least 100,000. In some particular aspects of the invention dealing with active immunization, mannuronic acid has a length of less than 1000 monomers in length. Mannuronic acid, as used herein, does not embrace native alginate. As used herein, native alginate is naturally occurring alginate or an alginate that is identical in structure and sequence to naturally occurring alginate.

FIGs. 1 and 2 illustrate the structure of two beta-1,4 linked D-mannuronic acid monomers. However, it is to be understood that the invention embraces both L and D isomers of mannuronic acid.

As used herein, a beta-1,4 linked "homopolymer" of D-mannuronic acid is an oligomer or polymer that comprises at least 4 D-mannuronic acid monomers. It may comprise 5, 6, 7, 8, 9,10,11,12,13, 14, 15, 16, 17, 18, 19, 20, 25, 50, 75, 100, or more (and every integer therebetween as if explicitly recited herein). A similar definition would apply to a homopolymer of L-mannuronic acid. Beta-1,4 linked homopolymer of D-mannuronic acid, as used herein, does not embrace native alginate.

In some aspects and embodiments, the mannuronic acid may be conjugated to one or more non-mannuronic acid residues. For example, mannuronic acid may be conjugated to one or more residues of guluronic acid, provided that the guluronic acid does not interfere with the activity of mannuronic acid in a particular method or composition. For example, in the screening methods provided herein, in some instances it may be possible to use a mannuronic acid oligomer or polymer that is conjugated to one or more guluronic acid monomers provided that the presence of guluronic acid does not result in selection of binding partners to mannuronic acid at a frequency which would occur if native alginate was used as the screening tool. Thus, for example, an oligomer or a polymer containing at least 55%, at least 60%, at least 65%, at least 70%, at least 75%, at least 80%, at least 85%, at least 90%, at least 95 %, or at least 99% mannuronic acid may be used as the screening tool in place of pure mannuronic acid, in some embodiments. In other related embodiments, the remaining portion of the polymer is guluronic acid. In some embodiments, an oligomer or polymer comprised of mannuronic acid - guluronic acid repeats is used.

Mannuronic acid may be conjugated to other non-mannuronic acid residues such as but not limited to D-lyxuronic acid, alpha-D-galacturonic acid, beta-D-galacturonic acid, beta-D-glucuronic acid, D-glucuronic acid, 3-O-methyl D-glucuronic acid, 4-O-methyl L-gulurono-3, 6-lactone, and L-iduronic acid. In some embodiments, an oligomer or polymer that contains at least one (and preferably more) mannuronic acid 4-mer (i.e., 4 contiguous mannuronic acid monomers), regardless of whether it further contains other residues, may be used. In preferred embodiments, however, the methods and compositions of the invention use mannuronic acid that is not conjugated to other monomers or residues.

As described herein, mannuronic acid may be conjugated to other moieties such as detectable labels (as described in greater detail herein) and carrier compounds (as described herein and in published PCT application WO 2004/043405, published on May 27, 2004, the entire content of which is incorporated herein in its entirety).

The mannuronic acid may be acetylated or not acetylated. The degree of acetylation may be less than 5%, less than 10%, less than 15%, less than 20%, less than 25%, less than 30%, less than 35%, less than 40%, and less than 45%, depending on the embodiment and the intended use. As an example of a suitable polymer for use in immunization may be, for example, a polymer having at least 75% mannuronic acid content and less than 45% acetylation.

In some instances, mannuronic acid is not derivatized at a hydroxyl group (i.e., it is not O-derivatized) meaning that it is has not been chemically manipulated to artificially change the number and kind of substituents attached to hydroxyl groups.

Mannuronic acid can be isolated or derived from naturally occurring sources or it may be synthesized de novo. Mannuronic acid can be isolated from naturally occurring sources as mannuronic acid homopolymer or derived from native alginate (e.g., by cleaving and isolating mannuronic acid monomer, oligomer or polymer from alginate). Mannuronic acid oligomer or polymer is also referred to herein as polymannuronic acid. Naturally occurring sources of polymannuronic acid and/or mannuronic acid enriched alginate, including seaweed and bacterial sources, are known. For example, Chitnis et al. (21) produced a strain of mucoid *P*. *aeruginosa* mutant at the algg gene that only makes polymannuronic acid. Since *P. syringae* makes alginate using genes very similar to those of *P*. *aeruginosa* (22), inactivation of the *algg* gene in *P. syringae* should also yield the same polymannuronic acid producing phenotype. As another example, *A. vinelandii* initially synthesizes polymannuronic acid and then introduces guluronic acid residues therein by an extracellular guluronic acid epimerase (referred to as a C-5 epimerase). Inactivation of C-5 epimerase can result in production of only polymannuronic acid by this organism. The same is true for seaweed (Madgwick et al., Acta Chem Scand. 1973;27(9):3592-4). Alginate from seaweed that is 80-90% mannuronic acid is also commercially available.

An isolated oligomer, polymer or Homopolymer, as used herein, is an oligomer, polymer or homopolymer that has been physically separated from the molecules or cellular components with which it would normally be associated in nature. For example, an isolated beta-1,4 linked homopolymer of D-mannuronic acid excludes intact bacteria or bacterial cell wall and cell membrane fractions. Isolated mannuronic acid or an isolated epitope thereof is also physically separated from native alginate.

Alginate can be isolated from various sources including bacteria such as but not limited to *Pseudomonas* spp. and *Azotobacter* spp. Examples of *Pseudomonas* spp. include but are not limited to *P. aeruginosa, P. syringae P. fluorescens, P. putida* and *P. mendocina.* The *Pseudomonas* spp. can be a mucoid or a non-mucoid strain. (See Sherbrock-Cox et al. Carbohydrate Research 1984 13S(1):147-154 for a method for purifying alginate.) Previously, a mucoid strain was defined as a strain that produces alginate and a non-mucoid strain was defined as a strain that did not. However, it has been discovered according to the invention that most "non-mucoid" strains make low amounts of alginate in vitro, and a few that do not can still be shown to make alginate in vivo. An example of an *Azotobacter* spp. is *Azotobacter vinelandii.* Other sources of alginate include plants such as but not limited to seaweed (e.g., *L. hyperborea).*

Mannuronic acid or an epitope containing fragment thereof may be used as an antigen for inducing active immunization in a subject. As used herein, an antigen is a molecule or molecules capable of stimulating in a subject an immune response that may include both cell-mediated immunity and humoral immunity. Preferably, the antigen is formulated as a vaccine.

As stated above, mannuronic acid or an epitope containing fragment thereof may be conjugated to a carrier compound, either directly or via a linker. The conjugation can occur at any position in the mannuronic acid monomer unit or at the ends of the oligomer or polymer.

A "carrier compound" as used herein is a compound that can be conjugated to a polysaccharide either directly or through the use of a linker and that may be immunologically active or inert. Carrier compounds include but are not limited to proteins, or peptides, polysaccharides, nucleic acids, or other polymers, lipids and small molecules. Proteins include, for example, plasma proteins such as serum albumin, immunoglobulins, apolipoproteins and transferrin, bovine thyroglobulin, keyhole limpet hemocyanin, bacterial polypeptides such as TRPLE, & galactosidase, polypeptides such as herpes gD protein, allergens, diphtheria and tetanus toxoids, salmonella flagellim, hemophilus pilin, hemophilus 15kDa, 28-30kDa and 40kDa membrane proteins, *E*. *coli* heat label enterotoxin ltb, cholera toxin, soy bean trypsin inhibitor, *P. aeruginosa* exotoxin A, and viral proteins including rotavirus VP and respiratory syncytial virus f and g proteins. The proteins useful in the invention include any protein that is safe for administration to mammals and optionally that is an immunologically effective carrier protein.

Many methods are known in the art for conjugating a polysaccharide to a protein. In general, the polysaccharide should be activated or otherwise rendered amenable to conjugation, i.e., at least one moiety must be rendered capable of covalently bonding to a protein or other molecule. Many such methods are known in the art. See for example U.S. Patent No. 4,356,170; U.S. Patent No. 4,619,828; U.S. Patent No. 4,663,160; U.S. Patent No. 4,808,700; and U.S. Patent No. 4,711,779. Many other methods of conjugation are known in the art.

The carrier compound may be conjugated to mannuronic acid or an epitope containing fragment thereof through a linker or spacer by any means known in the art. For example, a free reducing end of the polysaccharide may be used to produce a covalent bond with a spacer or linker. A covalent bond may be produced by converting a free reducing end of mannuronic acid into a free 1-aminoglycoside, that can subsequently be covalently linked to a spacer by acylation. (Lundquist et al., J Carbohydrate Chem., 10:377 (1991)). Alternatively, mannuronic acid may be covalently linked to the spacer using an N-hydroxysuccinimide active ester as activated group on the spacer. (Kochetkow, Carbohydrate Research, 146:C1 (1986)). The free reducing end of mannuronic acid may also be converted to a lactone using iodine and potassium hydroxide. (Isebell et al., Methods of Carbohydrate Chemistry, Academic Press, New York (1962)). The lactone can be covalently linked to the spacer by means of a primary amino group on the spacer or linker. The free reducing end of mannuronic acid may also be covalently linked to the linker or spacer using reductive amination. Other methods include use of cyanylating reagents to activate the free hydroxyl groups, or limited activation of the carboxylate groups with water soluble carbodiimide onto which a variety or reactive groups can be placed (sulphydryl, amino, etc.). Numerous methods applicable to making polysaccharide conjugates are known in the art (23).

The invention provides methods for identifying binding partners for mannuronic acid, and particularly for the carboxylate group on C6 of mannuronic acid. These binding partners may be peptide or non-peptide in nature. For example, they can be comprised of naturally and non-naturally occurring amino acids, peptidomimetic moieties, carbohydrates, nucleotides, lipids, and the like. They may derive from naturally occurring or synthetic sources. An example of a naturally occurring source is a cell extract, a tissue homogenate, or ascites fluid. An example of a synthetic source is a synthetic library, such as but not limited to an antibody library. For the sake of brevity and clarity, the invention refers primarily to binding peptides as an exemplary class of binding partners; however, this is not intended to limit the scope of the invention and should not be so construed. As used herein, a "candidate" binding partner (such as a candidate peptide) is a molecule that is being tested for its ability to bind to mannuronic acid or an epitope containing fragment thereof. Binding partners specifically exclude polycations such as poly-L-lysine and poly-D-lysine and epimerase.

In some instances, it may be possible to synthesize and screen candidate peptides using one system such as for example a phage display screening system as described in Hart, et al., J Biol. Chem. 269:12468 (1994). In general, phage display libraries using, e.g., M13 or fd phage, are prepared using conventional procedures such as those described in the foregoing reference. The libraries display inserts containing from 4 to 80 amino acid residues. The inserts optionally represent a completely degenerate or a biased array of peptides. Ligands that bind selectively to mannuronic acid are obtained by selecting phage that express on their surface a ligand that binds to mannuronic acid. These phage then are subjected to several cycles of reselection to identify the ligand-expressing phage that have the most useful binding characteristics. Typically, phage that exhibit the best binding characteristics (e.g., highest affinity) are further characterized by nucleic acid analysis to identify the particular amino acid sequences of the peptides expressed on the phage surface and the optimum length of the expressed peptide to achieve optimum binding to mannuronic acid.

Alternatively, such peptide ligands can be selected from combinatorial libraries of, for example, peptides containing one or more amino acids. Other libraries can be synthesized which contain non-peptide synthetic moieties and which are thus less susceptible to enzymatic degradation compared to their naturally occurring counterparts.

Additionally small peptides may easily be synthesized or produced by recombinant means to produce the peptide of the invention. Such methods are well known to those of ordinary skill in the art. Peptides can be synthesized for example, using automated peptide synthesizers which are commercially available. The peptides can be produced by recombinant techniques by incorporating the DNA expressing the peptide into an expression vector and transforming cells with the expression vector to produce the peptide.

The screening methods of the invention can be binding assays that discriminate between compounds that bind to mannuronic acid or an epitope containing fragment thereof and those that don't. The screening methods can be competition assays that identify compounds based on their ability to disrupt an antigen-antibody complex or to prevent such a complex from forming substantially. In this instance, the antigen is a compound that minimally comprises the carboxylate group on C6 of mannuronic acid, and the antibody is mAb F428 or mAb F429, or an antigen binding fragment thereof. Antibody fragments will be discussed in greater detail herein. As used herein, the term "mAb F428" refers to a monoclonal antibody having the binding specificity of mAb F428γ1 regardless of its isotype background, unless otherwise specified. Thus, for example, mAb F428 can be of an IgG1, IgG3 or IgG4 isotype. In some aspects and embodiments IgG1, IgG3 and IgG2 are preferred, and in other aspects and embodiments. IgG1 and IgG3 are preferred. The foregoing applies equally to mAb F429.

Standard binding assays are well known in the art, and a number of these are suitable in the present invention including ELISA, competition binding assay, sandwich assays, radioreceptor assays using radioactively labeled peptides or radiolabeled antibodies, immunoassays, etc. The nature of the assay is not essential provided it is sufficiently sensitive to detect binding of a small number of peptides.

In one aspect, the screening method simply determines whether a candidate binding partner (such as a candidate peptide) is able to bind to mannuronic acid, and more preferably the extent of its binding by comparison to a control. Candidate molecules that bind to mannuronic acid to a meaningful degree as described herein are referred to as mannuronic acid binding partners. Similarly, candidate peptides that bind to mannuronic acid to a meaningful degree as described herein are referred to as mannuronic acid binding peptides. As used herein, a mannuronic binding peptide is a peptide that binds selectively to mannuronic acid or epitope-containing fragments thereof. As used herein, the terms "selective binding" and "specific binding" are used interchangeably to refer to the ability of the peptide to bind with greater affinity to mannuronic acid and epitope-containing fragments thereof than to non-mannuronic acid and non-epitope-containing compounds. That is, peptides that bind selectively to mannuronic acid will not bind to non-mannuronic acid derived compounds (that do not structurally contain the same epitope) to the same extent and with the same affinity as they bind to mannuronic acid and epitope-containing fragments thereof. For example, a selectively binding peptide may bind to mannuronic acid with an affinity that is at least 2-fold, at least 3-fold, at least 4-fold, at least 5-fold, at least 10-fold, at least 25-fold, at least 50-fold, at least 100-fold (or more) greater than its affinity for a non-mannuronic acid moiety. In some embodiments, the peptides bind solely to mannuronic acid and epitope containing fragments thereof (i.e., the level of binding to other compounds that do not comprise mannuronic acid or the epitope thereof is negligible). The terms "mannuronic acid binding peptide" and "mannuronic acid specific binding peptide" are used interchangeably herein.

The method may involve the incubation of a candidate peptide together with a mannuronic acid homopolymer such as a beta-1,4 linked homopolymer of D-mannuronic acid followed by a determination of a test level of binding between the two components. That test level of binding is then compared to a control to determine whether the candidate peptide is a mannuronic acid binding peptide. The control can be a negative control or a positive control. In some embodiments, the test level of binding may be compared to both a negative and a positive control. The control level of binding, regardless of whether it is a negative or positive control, can be obtained from an assay run alongside the test binding assay (or before or thereafter), or from a database of positive and negative control levels of binding from previously run assays. The screening assay can similarly be accomplished using a polymer that comprises at least one stretch of 4 or more contiguous mannuronic acid monomers, regardless of its remaining content. In this latter embodiment, the polymer is preferably not native alginate.

The negative control level of binding may be determined by measuring the level of binding between the mannuronic acid and an empty vehicle (e.g., a solution identical to that in which the candidate peptide is presented but lacking the candidate peptide), or between the candidate peptide and an empty vehicle (e.g., a solution identical to that in which the mannuronic acid is presented but lacking mannuronic acid). The negative control level of binding alternatively may be the level of binding between the mannuronic acid and a peptide that is known not to bind to mannuronic acid or between the candidate peptide and mannose. Mannose differs from mannuronic acid in that mannose lacks the carboxylic acid (or carboxylate group) on C6.

Generally a test level of binding that is greater than a negative control level of binding is indicative of a mannuronic acid binding peptide. In some instances, the test level of binding may be at least 2-fold, at least 3-fold, at least 4-fold, at least 5-fold, at least 10-fold, at least 20-fold, at least 50-fold, or at least 100-fold greater than the negative control level of binding.

The positive control level of binding may be determined by measuring the level of binding between the mannuronic acid and a peptide known to bind to mannuronic acid such as for example an antigen-binding fragment of mAb F428 or mAb F429 or in some instances the intact mAb itself. Wherever possible, it may be advisable to parallel positive and negative control components with those used in the test binding assay (e.g., if the candidate peptide is a monoclonal antibody, then the positive control may use mAb F428 or mAb F429).

Generally a test level of binding that is substantially equal to or greater than the positive control level of binding is indicative of a mannuronic acid binding peptide. A "substantially equal" level of binding is a level of binding that is within +/- 25% of the positive control level of binding. In some instances, the level of binding is within +/- 20%, within +/- 15%, within +/- 10%, within +/- 5%, or within +/- 1 % of the positive control level of binding.

Another example of a binding assay is a competition assay in which the candidate peptide is characterized based on its ability to preclude or reduce binding of mannuronic acid with mAb F428 or mAb F429, or an antigen-binding fragment thereof. The level of binding of the monoclonal antibody to mannuronic acid in the presence or absence of the candidate peptide is determined. In this assay, the monoclonal antibody (or a fragment thereof) can be incubated with mannuronic acid first followed by addition of the candidate peptide. The level of binding can be determined in the same reaction vessel consecutively (i.e., before and after the addition of the candidate peptide). Alternatively, all three components can be added to the reaction vessel together. This latter configuration would require a separate determination of the level of binding of the monoclonal antibody to mannuronic acid.

MAb F429 has been deposited with the ATCC on August 10, 2004, and has been assigned Accession No. PTA-6155. MAb F428 is comprised of a heavy chain variable region identical to that of F429 and a light chain variable region identical to that of mAb F431 which has also been deposited with the ATCC on August 10, 2004, and has been assigned Accession No. PTA-6156. These sequences are disclosed in U.S. Patent Application No. 10/440,522, filed May 16, 2003, and have also been deposited with GenBank, as described in greater detail in the Examples. One of ordinary skill in the art will be able to make both mAb F428 and F429 (of various isotypes), based on this information and the level of skill in the art.

The screening assays described herein can be overlapped with each other (i.e., both assays may be applied to all or some candidate peptides). Additionally or alternatively, other assays may be used either before or after these binding assays, for example either as pre-screens or as confirmatory assays. For example, an appropriate prescreen would be an assay that identifies and eliminates binding candidates that bind to guluronic acid. Another example of a prescreen is an assay to identify and eliminate candidate peptides that bind to mAb F428 and F429. Another prescreen is an assay to identify and eliminate candidate peptides that bind to mannose. An example of a confirmatory assay is one in which a mannuronic acid binding peptide is tested in an in vitro assay (e.g., a cell killing (opsonophagocytic) assay) or an in vivo assay (e.g., an immune response induction assay).

Thus, binding peptides that are antibodies or Fc containing fragments thereof can be further screened for their ability to enhance opsonization and phagocytosis (i.e., opsonophagocytosis) of *P. aeruginosa* or other alginate producing organism. Phagocytosis refers to the process by which phagocytic cells (e.g., macrophages, dendritic cells, and polymorphonuclear leukocytes (PMNL)) engulf material and enclose it within a vacuole (e.g., a phagosome) in their cytoplasm. Opsonization refers to a process by which phagocytosis is facilitated by the deposition of opsonins (e.g., antibody or complement factors such as C3b) onto the antigen. Thus, antibodies or antibody fragments that enhance opsonization and phagocytosis are antibodies or antibody fragments that recognize and bind to an antigen, and in doing so, facilitate the uptake and engulfment of the antigen (and the antigen-bearing substance, e.g., *P. aeruginosa* bacteria) by phagocytic cells. Generally, in order to enhance phagocytosis and opsonization, the antibody comprises an Fc domain or region. The Fc domain is recognized by Fc receptor bearing cells (e.g., antigen presenting cells such as macrophages, or PMNL). As used herein, "to enhance opsonophagocytosis" means to increase the likelihood that an antigen or an antigen bearing substrate will be recognized and engulfed by a phagocytic cell, via antibody deposition. This enhancement can be measured inter alia by reduction in bacterial load in vivo or by bacterial cell killing in vitro using the in vitro methods described below.

Opsonization assays are standard in the art. Generally such assays measure the amount of bacterial killing in the presence of an antibody, an antigen (expressed on the target bacterial cell), complement, and phagocytic cells. Serum is commonly used as a source of complement, and polymorphonuclear cells are commonly used as a source of phagocytic cells. The target cell source can be prokaryotic (as in the present invention) or eukaryotic, depending upon which cell type expresses the antigen. Cell-killing can be measured by viable cell counts prior to and following incubation of the reaction components. Alternatively, cell killing can be quantitated by measuring labeled cell contents in the supernatant of the reaction mixture (i.e., chromium release). Other assays will be apparent to those of skill in the art, having read the present specification, which are useful for determining whether an antibody or antibody fragment that binds to mannuronic acid or an epitope containing fragment thereof also stimulates opsonization and phagocytosis.

A variety of other reagents also can be included in the binding mixture. These include reagents such as salts, buffers, neutral proteins (e.g., albumin), detergents, etc. which may be used to facilitate optimal binding. Such a reagent may also reduce non-specific or background interactions of the reaction components. Other reagents that improve the efficiency of the assay may also be used. The mixture of the foregoing assay materials is incubated under conditions under which for example mAb F428 or mAb F429 selectively binds to mannuronic acid. Such conditions will preferably mimic physiological conditions. The order of addition of components, incubation temperature, time of incubation, and other parameters of the assay may be readily determined. Such experimentation merely involves optimization of the assay parameters, not the fundamental composition of the assay. Incubation temperatures typically are between 4°C and 40°C. Incubation times preferably are minimized to facilitate rapid, high throughput screening, and typically are between 0.1 and 10 hours. After incubation, the presence or absence of selective binding is detected by any convenient method available to the user. Typically, a plurality of assay mixtures are run in parallel with different peptides or different peptide concentrations.

A separation step is often used to separate bound from unbound components. The separation step may be accomplished in a variety of ways. Usually one of the components is bound or fixed to a solid support, and its binding partner by definition also appears bound to the solid support by virtue of the interaction between the two components. Unbound components (i.e., components that are not binding partners) may be easily separated from the bound components for example by rinsing or washing the reaction vessel. The solid substrate can be made of a wide variety of materials and in a wide variety of shapes, e.g., columns or gels of polyacrylamide, agarose or sepharose, microtiter plates, microbeads, magnetic beads, resin particles, etc. For example, when the solid substrate is a microtiter plate, the wells may be washed several times with a washing solution, which typically includes those components of the incubation mixture that do not participate in specific bindings such as salts, buffer, detergent, non-specific protein, etc. Where the solid substrate is a magnetic bead, the beads may be washed one or more times with a washing solution and isolated using a magnet.

The invention also contemplates assays in which neither component is fixed, as there are ways of detecting a binding event without removing unbound components, as will be discussed herein.

Typically, at least one of the components usually comprises, or is coupled or conjugated to a detectable label. A detectable label is a moiety, the presence of which can be ascertained directly or indirectly. Generally, detection of the label involves an emission of energy by the label. The label can be detected directly for example by its ability to emit and/or absorb electromagnetic radiation of a particular wavelength. A label can be detected indirectly by its ability to bind, recruit and, in some cases, cleave another moiety which itself may emit or absorb light of a particular wavelength (e.g., an epitope tag such as the FLAG epitope, an enzyme tag such as horseradish peroxidase, etc.).

Generally, a detectable label can be but is not limited to a chromogenic molecule, a fluorescent molecule (e.g., fluorescein isothiocyanate (FITC), TRITC, rhodamine, tetramethylrhodamine, R-phycoerythrin, Cy-3, Cy-5, Cy-7, Texas Red, Phar-Red and allophycocyanin (APC)), a chemiluminescent molecule, a bioluminescent molecule, a radioisotope (e.g., P³² or H³, ¹⁴C, ¹²⁵I and ¹³¹I), an optical or electron density molecule, an electromagnetic molecule, an electrical charge transducing or transferring molecule, a semiconductor nanocrystal or nanoparticle, an electron spin resonance molecule (such as for example nitroxyl radicals), a nuclear magnetic resonance molecule, a colloidal metal, a colloid gold nanocrystal, a microbead, a magnetic bead, a paramagnetic particle, a quantum dot, an enzyme (e.g., alkaline phosphatase, horseradish peroxidase, β-galactosidase, glucoamylase, lysozyme, luciferases such as firefly luciferase and bacterial luciferase (U.S. Patent No. 4,737,456); saccharide oxidases such as glucose oxidase, galactose oxidase, and glucose-6-phosphate dehydrogenase; heterocyclic oxidases such as uricase and xanthine oxidase coupled to an enzyme that uses hydrogen peroxide to oxidize a dye precursor such as HRP, lactoperoxidase, or microperoxidase), an enzyme substrate, an affinity molecule, a ligand, a receptor, a biotin molecule, an avidin molecule, a streptavidin molecule, an antigen (e.g., epitope tags such as the FLAG or HA epitope), a hapten (e.g., biotin, pyridoxal, digoxigenin fluorescein and dinitrophenol), an antibody and an antibody fragment. The label may be of a chemical, lipid, carbohydrate, peptide or nucleic acid nature although it is not so limited. Those of ordinary skill in the art will know of other suitable labels for the binding assay components (or therapeutic agents described herein), or will be able to ascertain such information using routine experimentation.

The detection system can be selected from any number of detection systems known in the art. These include a charge coupled device (CCD) detection system, an electron spin resonance (ESR) detection system, an electrical detection system, an electron microscopy detection system, a confocal laser microscopy detection system, a photographic film detection system, a fluorescent detection system, a chemiluminescent detection system, an enzyme detection system, an atomic force microscopy (AFM) detection system, a scanning tunneling microscopy (STM) detection system, a scanning electron microscopy detection system, an optical detection system, an electron density detection system, a refractive index system, a nuclear magnetic resonance (NMR) detection system, a near field detection system, a total internal reflection (TIR) detection system, and a electromagnetic detection system.

The label may be bound to a reaction component during or following its synthesis. As used herein, "conjugated" means two entities stably bound to one another by any physiochemical means. It is important that the nature of the attachment is such that it does not substantially impair the effectiveness of either entity. Keeping these parameters in mind, any covalent or non-covalent linkage known to those of ordinary skill in the art may be employed. In some embodiments, covalent linkage is preferred. Noncovalent conjugation includes hydrophobic interactions, ionic interactions, high affinity interactions such as biotin-avidin and biotin-streptavidin complexation and other affinity interactions. Such means and methods of attachment are known to those of ordinary skill in the art. Furthermore, the coupling or conjugation of these labels to the binding assay components of the invention can be performed using standard techniques common to those of ordinary skill in the art. For example, U.S. Patent Nos. 3,940,475 and 3,645,090 demonstrate conjugation of fluorophores and enzymes to antibodies.

In some embodiments, more than one component in the reaction mixture is labeled and the close association of those components (due to specific binding) results in the decrease or absence or the increase or presence of a signal. One common example of this configuration is fluorescence resonance energy transfer (FRET). In FRET, one component is labeled with an donor molecule that accepts light of a certain wavelength and emits light of another. A second component is labeled with an acceptor molecule that accepts light at the wavelength emitted by the donor molecule and emits light at a different wavelength. The donor molecule however can only impart its emitted light if it is in close enough proximity to the acceptor molecule, which can occur if they are bound to each other. The binding of two FRET labeled components therefore is indicated by the emission of light from the acceptor molecule. Lack of binding or disruption of that binding however is indicated by the decrease or absence of such emission. FRET analysis is particularly useful when none of the components is fixed or immobilized to a solid support. It does require however labeling of at least two components and therefore can be more expensive than a single labeled component assay.

A FRET fluorophore pair is two fluorophores that are capable of undergoing FRET to produce, increase, eliminate or reduce a detectable signal when positioned in proximity to one another. Examples of donors include Alexa 488, Alexa 546, BODIPY 493, Oyster 556, Fluor (FAM), Cy3 and TMR (Tamra). Examples of acceptors include Cy5, Alexa 594, Alexa 647 and Oyster 656. Cy5 can work as a donor with Cy3, TMR or Alexa 546, as an example. FRET should be possible with any fluorophore pair having fluorescence maxima spaced about 50-100 nm from each other.

As an example of a binding assay, mannuronic acid can be immobilized on a surface (such as in a well of a multi-well plate) and then contacted with a labeled candidate peptide. The amount of peptide that binds to mannuronic acid (and thus becomes itself immobilized onto the surface) may then be quantitated to determine whether the candidate peptide binds to mannuronic acid. Alternatively, the amount of peptide not bound to the surface may also be measured.

Peptide binding can also be tested using a competition assay. If the candidate peptide competes with the monoclonal antibodies or antibody fragments described herein, as shown by a decrease in binding of the monoclonal antibody or fragment to mannuronic acid, then it is likely that the peptide and the monoclonal antibody bind to the same or at least an overlapping epitope, provided that the peptide does not bind to the monoclonal antibody. In this assay system, the antibody or antibody fragment may be labeled and the mannuronic acid may be immobilized onto the solid surface. Alternatively, the monoclonal antibody and mannuronic acid may be labeled with a FRET donor/acceptor pair, and the decrease or loss of acceptor signal or emission indicates disruption of binding by the candidate peptide.

As used herein, the term "peptide" includes monoclonal antibodies, functionally active and/or equivalent antibody fragments, and functionally active and/or equivalent peptides and polypeptides. The peptides of the invention are preferably isolated peptides. As used herein, "isolated peptides" are peptides that are substantially pure and are essentially free of other substances with which they may be found in nature or *in vivo* systems to an extent practical and appropriate for their intended use. In particular, the peptides are sufficiently pure and are sufficiently free from other biological constituents of their hosts cells so as to be useful in, for example, producing pharmaceutical preparations or sequencing. Because an isolated peptide of the invention may be admixed with a pharmaceutically acceptable carrier in a pharmaceutical preparation, the peptide may comprise only a small percentage by weight of the preparation. The peptide is nonetheless isolated in that it has been substantially separated from the substances with which it may be associated in living systems.

Once a binding peptide is identified, the invention intends to embrace conservative substitutions of that binding peptide. As used herein, "conservative substitution" refers to an amino acid substitution which does not alter the relative charge or size characteristics of the peptide in which the amino acid substitution is made. Conservative substitutions of amino acids include substitutions made amongst amino acids with the following groups: (1) M,I,L,V; (2) F,Y,W; (3) K,R,H; (4) A,G; (5) S,T; (6) Q,N; and, (7) E,D.

Such substitutions can be made by a variety of methods known to one of ordinary skill in the art. For example, amino acid substitutions may be made by PCR-directed mutation, site-directed mutagenesis according to the method of Kunkel (Kunkel, Proc. Nat. Acad. Sci. U.S.A. 82: 488-492, 1985), or by chemical synthesis of a gene encoding the particular CDR. These and other methods for altering a peptide will be known to those of ordinary skill in the art and may be found in references which compile such methods, e.g. Sambrook or Ausubel, noted above. In some embodiments, however, due to size, it may be more convenient to synthesize the variant peptides using a peptide synthesizer such as those commercially available.

Conservative substitutions can also be made in mAb F428 and F429 provided that the resultant mAb or fragments thereof continue to exhibit selective binding to mannuronic acid or an epitope containing fragment thereof, as described herein. Such antibody variants can be used in the competition binding assays of the invention.

The invention embraces binding peptides that are antibodies. It also contemplates the use of particular antibodies and antigen binding fragments thereof in competition binding assays. An antibody, as is known in the art, is an assembly of polypeptide chains linked by disulfide bridges. Two principle polypeptide chains, referred to as the light chain and heavy chain, make up all major structural classes (isotypes) of antibody. Both heavy chains and light chains are further divided into subregions referred to as variable regions and constant regions. In some instances, the peptides encompass the antibody heavy and light variable chains of the foregoing antibodies. The heavy chain variable region is a peptide which generally ranges from 100 to 150 amino acids in length. The light chain variable region is a peptide which generally ranges from 80 to 130 amino acids in length.

As is known in the art, CDRs of an antibody are the portions of the antibody which are largely responsible for antibody specificity. The CDRs directly interact with the epitope of the antigen (see, in general, Clark, 1986; Roitt, 1991). In both the heavy chain and the light chain variable regions of IgG immunoglobulins, there are four framework regions (FR1 through FR4) separated respectively by three complementarity determining regions (CDR1, CDR 2 and CDR3). The framework regions (FRs) maintain the tertiary structure of the paratope, which is the portion of the antibody which is involved in the interaction with the antigen. CDRs, and in particular CDR3, and more particularly heavy chain CDR3, contribute to antibody specificity. Because CDRs, and in particular CDR3, confer antigen specificity on the antibody, these regions may be incorporated into other antibodies or peptides to confer the identical antigen specificity onto that antibody or peptide.

In other embodiments, the binding peptide is an antibody fragment such as but not limited to Fab, Fc, pFc', F(ab')₂, Fd and Fv. These terms are employed with standard immunological meanings [Klein, Immunology (John Wiley, New York, NY, 1982); Clark, W.R. (1986) The Experimental Foundations of Modern Immunology (Wiley & Sons, Inc., New York); Roitt, I. (1991) Essential Immunology, 7th Ed., (Blackwell Scientific Publications, Oxford)]. As is known in the art, only a portion of an antibody molecule is involved in binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions of the antibody, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. An isolated F(ab')₂ fragment is referred to as a bivalent monoclonal fragment because of its two antigen binding sites. An Fab fragment retains one of the antigen binding sites of an intact antibody molecule. Fab fragments consist of a covalently bound antibody light chain and a portion of the antibody heavy chain denoted Fd (heavy chain variable region). The Fd fragments are the major determinant of antibody specificity (a single Fd fragment may be associated with up to ten different light chains without altering antibody specificity) and Fd fragments retain epitope-binding ability in isolation.

Antibody fragments also embrace single chain antibodies. Single chain antibodies and fragments thereof (ScFv) can be constructed for example in accordance with the methods described in U.S. Patent No. 4,946,778 to Ladner et al. Such single chain antibodies include the variable regions of the light and heavy chains joined by a flexible linker moiety. Methods for obtaining a single domain antibody ("Fd") which comprises an isolated variable heavy chain single domain, also have been reported (see, for example, Ward et al., Nature 341:644-646 (1989), disclosing a method of screening to identify an antibody heavy chain variable region (V_{H} single domain antibody) with sufficient affinity for its target epitope to bind thereto in isolated form). Methods for making recombinant Fv fragments based on known antibody heavy chain and light chain variable region sequences are known in the art and have been described, e.g., Moore et al., US Patent No. 4,462,334. Other references describing the use and generation of antibody fragments include e.g., Fab fragments (Tijssen, Practice and Theory of Enzyme Immunoassays (Elsevieer, Amsterdam, 1985)), Fv fragments (Hochman et al., Biochemistry 12: 1130 (1973); Sharon et al., Biochemistry 15: 1591 (1976); Ehrilch et al., U.S. Patent No. 4,355,023) and portions of antibody molecules (Audilore-Hargreaves, U.S. patent No. 4,470,925). Thus, those skilled in the art may construct antibody fragments from various portions of intact antibodies without destroying the specificity of the antibodies for the antigen.

Depending upon the aspect and embodiment of the invention, the antibody may be a monoclonal antibody or a polyclonal antibody. As used herein, the term "monoclonal antibody" refers to a homogenous population of immunoglobulins that specifically bind to an identical epitope (i.e., antigenic determinant). Monoclonal antibodies have the same Ig gene rearrangement and thus demonstrate identical binding specificity. Polyclonal antibodies are a heterogeneous population of immunoglobulins that bind to various epitopes and in some instances various antigens.

Monoclonal antibodies can be prepared by any method known in the art such as by immortalizing spleen cells isolated from the immunized animal by e.g., fusion with myeloma cells or by Epstein Barr Virus transformation, and screening for clones expressing the desired antibody. Other methods involve isolation of rearranged Ig gene sequences and cloning into immortalized cell lines. Methods for preparing and using monoclonal antibodies are well known in the art.

Murine monoclonal antibodies may be made by any of these methods utilizing the desired antigen as an immunogen. The following description of a method for developing an anti-mannuronic acid monoclonal antibody is exemplary and is provided for illustrative purposes only. Balb/c mice are immunized intraperitoneally with approximately 75-100 µg of mannuronic acid (as antigen) in complete Freund's adjuvant. Booster injections of approximately 25-50 µg antigen in incomplete Freund's are administered on approximately days 15 and 35 after the initial injection. On day 60-65, the mice receive booster injections of approximately 25 µg antigen in the absence of adjuvant. Three days later, the mice are killed and the isolated spleen cells fused to murine myeloma NS-1 cells using polyethylene glycol by a procedure such as that described by Oi (Oi VT: Immunoglobulin-producing hybrid cell lines in Herzenberg LA (ed): Selected Methods in Cellular Biology, San Francisco, CA, Freeman, (1980)). Hybridoma cells are selected using hypoxanthine, aminopterin, and thymidine (HAT) and grown in culture. Fourteen to fifteen days after fusion, hybridoma cells producing anti-mannuronic acid monoclonal antibodies are identified using a solid-phase radioimmunoassay by capturing anti-mannuronic acid antibodies from conditioned media with immobilized goat anti-mouse IgG followed by quantitation of specifically bound labeled mannuronic acid. Hybridomas testing positive for antibodies against mannuronic acid are subcloned by limiting dilution and re-tested. Ascites for the hybridomas is then prepared in pristane-primed BALB/c mice by injecting approximately 1 x 10⁶ cells/mouse. Concentrates enriched in the selected monoclonal antibodies are produced from ascites fluid by gel filtration on S-200 and concentrated with NH₄SO₄. The pellets are dissolved in an appropriate storage solution such as 50% glycerol/H₂O and are stored at 4°C.

A monoclonal antibody as used herein includes human and humanized monoclonal antibodies and fragments thereof. A human monoclonal antibody is an antibody that is comprises completely of human origin sequences. Human antibodies may be obtained by recovering antibody-producing lymphocytes from the blood or other tissues of humans. These lymphocytes can be treated to produce cells that grow on their own in the laboratory under appropriate culture conditions. The cell cultures can be screened for production of antibody to a particular antigen and then cloned. Clonal cultures can be used to produce human monoclonal antibodies, or the genetic elements encoding the variable portions of the heavy and light chain of the antibody can be cloned and inserted into nucleic acid vectors for production of antibody of different types.

Human monoclonal antibodies also may be made by any of the methods known in the art, such as those disclosed in US Patent No. 5,567,610, issued to Borrebaeck et al., US Patent No. 565,354, issued to Ostberg, US Patent No. 5,571,893, issued to Baker et al, Kozber, J. Immunol. 133: 3001 (1984), Brodeur, et al., Monoclonal Antibody Production Techniques and Applications, p. 51-63 (Marcel Dekker, Inc, New York, 1987), and Boerner et al., J. Immunol., 147: 86-95 (1991). In addition to the conventional methods for preparing human monoclonal antibodies, such antibodies may also be prepared by immunizing transgenic animals that are capable of producing human antibodies (e.g., Jakobovits et al., PNAS USA, 90: 2551 (1993), Jakobovits et al., Nature, 362: 255-258 (1993), Bruggermann et al., Year in Immunol, 7:33 (1993) and US Patent No. 5,569,825 issued to Lonberg).

A humanized monoclonal antibody as used herein is a human monoclonal antibody or functionally active fragment thereof having at least human constant regions and an antigen binding region (e.g., a CDR) from a mammal of a species other than a human. An intact humanized monoclonal antibody in an isolated form is particularly suited to some aspects of the invention. Humanized antibodies have particular clinical utility in that they specifically recognize an antigen, but will not evoke an immune response in humans against the antibody itself. A murine CDR may be grafted into the framework region of a human antibody. See, e.g., L. Riechmamn et al., Nature 332, 323 (1988); M. S. Neuberger et al., Nature 314, 268 (1985) and EPA 0 239 400 (published Sep. 30, 1987).

There are entities in the United States which will synthesize humanized antibodies from specific murine antibody regions commercially, such as Protein Design Labs (Mountain View California), Abgenix, and Medarex. Further examples are provided in European Patent Applications 0239400 and 0239400.

Antibody fragments also encompass human and humanized antibody fragments. As one skilled in the art will recognize, such fragments could be prepared by traditional enzymatic cleavage of intact human or humanized antibodies. If however intact antibodies are not susceptible to such cleavage because of the nature of their construction (e.g., as may be the case with humanized antibodies), the noted constructions can be prepared with immunoglobulin fragments used as the starting materials. Alternatively, if recombinant techniques are used, the DNA sequences themselves can be tailored to encode the desired fragment which when expressed can be combined *in vivo* or *in vitro*, by chemical or biological means, to prepare the final desired intact immunoglobulin fragment.

Polyclonal antibodies can be prepared by any method known in the art. For example, antigen is combined with an adjuvant such as Freund's complete adjuvant (e.g., 100 µg of conjugate for rabbits or mice in 1-3 volumes ofFreund's) and injected intradermally at multiple sites. Approximately one month later, the animals are boosted with 1/5 - 1/10 of the original amount of antigen or antigen conjugate, in adjuvant by subcutaneous injection at multiple sites. One to two weeks later the animals are bled, and the serum is assayed for the presence of antibody. The animals may be repeatedly boosted until the antibody titer plateaus. The animal may be boosted with antigen, with or without an adjuvant.

The invention also describes detection methods such as would be useful in diagnosis of infection. These methods can be performed in vivo or in vitro and generally involve contacting one or more binding partners of the invention with a sample in or from a subject. Preferably, the sample is first harvested from the subject, although in vivo detection methods are also envisioned. The sample may include any body tissue or fluid that is suspected of harboring an alginate producing organism. For example, a *P. aeruginosa* infection can affect any number of tissues including eye, ear, respiratory tract (including lung), heart (including heart valve), central nervous system, bone and joint, gastrointestinal tract (including large bowel), urinary tract, skin, and soft tissues. Lung or gastrointestinal lavages or CNS fluid can all be sampled and tested for the presence of the bacteria, for example. Those of ordinary skill in the art will be able to modify such detection assays based on the organism being detected and its target tissues.

In order to detect the alginate producing organism (e.g., the bacteria), the sample is contacted with a "mannuronic acid binding partner (e.g., binding peptide) of the invention and the level of binding between the two is compared to the level of binding to a sample that is known to be negative for the alginate producing organism, alginate, or mannuronic acid (i.e., a negative control). Binding partners that are conjugated to a detectable label are most useful in these assays. Suitable detectable labels and methods of conjugation are discussed herein.

The invention also describes methods of detecting alginate producing organisms (e.g., *P. aeruginosa*) in or on medical equipment, surfaces, instrumentation, and the like, in order to for example identify contamination by the organism (e.g., bacterial contamination). These detection methods are carried out essentially in the same manner as those described above. The items and surfaces to be tested are either contacted directly with the binding partners of the invention, or alternatively, they are sampled and the sample is tested for the presence of the organism. Sampling can include but is not limited to swabbing, wiping, flushing, and the like.

When used in vitro, the binding partners may be conjugated to the detectable labels discussed previously herein. When used in vivo, the binding partners may be conjugated to a number of labels including but not limited to diagnostic and imaging labels such as for magnetic resonance imaging (MRI): Gd(DOTA); for nuclear medicine: ²⁰¹Tl, gamma-emitting radionuclide 99mTc; for positron-emission tomography (PET): positron-emitting isotopes, (18)F-fluorodeoxyglucose ((18)FDG), (18)F-fluoride, copper-64, gadodiamide, and radioisotopes of Pb(II) such as 203Pb; 111In.

The compositions can also be used to determine the immunologic status of humans or animals with regard to their susceptibility to infections by alginate producing organisms.

The method further embraces treatment methods such as prophylactic and therapeutic methods. As used herein, the term "treatment" refers to the administration of antigens or binding partners to a subject for the purpose of achieving a medically desirable benefit. Accordingly, "treatment" intends to embrace both "prophylactic" and "therapeutic" treatment methods. Prophylactic treatment methods refer to treatment administered to a subject prior to diagnosis of an infection (or other condition or disease state associated with an alginate producing organism). In other words, the subject does not present with symptoms of either an infection or a related condition although the subject may be at risk of either or both. Subjects at risk include but are not limited to those that are cystic fibrosis (CF) patients (particularly early CF patients such as those that have yet to demonstrate existence of mucoid strains), burn patients, subjects undergoing mechanical ventilation, immunocompromised subjects, and the like. Therapeutic treatment methods refer to treatment administered to a subject after the diagnosis of an infection or a related condition. In other words, the subject has been diagnosed as having either an infection or a related condition or alternatively, the subject may exhibit symptoms associated with either or both.

As used herein, a subject includes a human, non-human primate, cow, horse, pig, sheep, goat, dog, cat or rodent. In some instances, as is discussed in greater detail herein, the subject is a non-rodent subject. A non-rodent subject is any subject as defined above, but specifically excluding rodents such as mice, rats, and rabbits. In all embodiments, human subjects are preferred.

The compositions of the invention are useful for active immunization of human or animals to prevent or treat infection by alginate producing organisms. Alginate producing organisms include but are not limited to *Pseudomonas* spp. such as *P. aeruginosa, P. fluorescens, P. putida* and *P. mendocina;* and *Azotobacter* spp. such as *Azotobacter vinelandii.* The antigen or vaccine compositions can also be used to produce anti-alginate or anti-mannuronic acid polyclonal antisera or polyclonal immunoglobulin that can be administered to other humans or animals to prevent or treat infections and related conditions.

Active immunization is accomplished by administering to a subject an effective amount for inducing an immune response such as an antibody response against alginate or mannuronic acid of any of the antigen or vaccine compositions of the invention. "Active immunity" as used herein involves the introduction of an antigen into a subject such that the antigen induces production antibody-producing cells and memory cells and various T cell populations. The active immunization methods provided herein specifically exclude immunization of subjects with intact or inactivated alginate producing bacteria or with purified naturally occurring alginate whether conjugated or not.

The mannuronic acid antigen may be formulated as a vaccine. A suitable carrier media for formulating a vaccine includes sodium phosphate-buffered saline (pH 7.4) or 0.125 M aluminum phosphate gel suspended in sodium phosphate-buffered saline at pH 6 and other conventional media. Generally, vaccines contain from about 5 to about 100 µg, and preferably about 10-50 µg of the antigen to elicit effective levels of antibody in warm-blooded mammals. When administered as a vaccine the antigen can optionally include an adjuvant. The term "adjuvant" is intended to include any substance which is incorporated into or administered simultaneously with the antigen and which potentiates the immune response in the subject. Adjuvants include but are not limited to aluminum compounds, e.g., gels, aluminum hydroxide and aluminum phosphate, and Freund's complete or incomplete adjuvant (e.g., in which the antigen is incorporated in the aqueous phase of a stabilized water in paraffin oil emulsion). The paraffin oil may be replaced with different types of oils, e.g., squalene or peanut oil. Other materials with adjuvant properties include BCG (attenuated *Mycobacterium tuberculosis*), calcium phosphate, levamisole, isoprinosine, polyanions (e.g., poly A:U), lentinan, pertussis toxin, lipid A, saponins, saponins such as QS-21, peptides, e.g. muramyl dipeptide, and immunostimulatory oligonucleotides such as CpG oligonucleotides. Rare earth salts, e.g., lanthanum and cerium, may also be used as adjuvants. The amount of adjuvants depends on the subject and the particular antigen used and can be readily determined by one skilled in the art without undue experimentation.

The antigen or vaccine compositions may be administered to any subject capable of generating an immune response to the antigen. A subject capable of generating an immune response to an antigen and at risk of developing an infection is a mammal possessing an immune system that is at risk of being exposed to environmental alginate producing organisms. For instance, hospitalized patients are at risk of developing *Pseudomonas* infection as a result of exposure to the bacteria in the hospital environment. Particular high risk populations for developing infection by *Pseudomonas* spp. include, for example, subjects having or at risk of developing cystic fibrosis, subjects having respiratory conditions, subjects requiring (and receiving) mechanical ventilation, subjects having ventilation-associated pneumonia, subject having cancer, and subjects that have been burned. Active immunization methods of the invention preferably are carried out in non-rodent subjects as defined herein and more preferably in human subjects. Mannuronic acid oligomer or polymer to be used in active immunization methods preferably has a length of less than 1000 monomers.

The antigen or vaccine composition can be administered to the subject in an effective amount for inducing an active immune response (e.g., an antibody response). An "effective amount for inducing an active immune response" as used herein is an amount of antigen which is sufficient to (i) assist the subject in producing its own immune protection by e.g. inducing the production of anti-alginate or anti-mannuronic acid antibodies in the subject, inducing the production of memory cells, and possibly a cytotoxic lymphocyte reaction etc., (ii) prevent infection by alginate producing organisms from occurring in a subject which is exposed to such organisms, (iii) inhibit the development of infection, for example, arresting or slowing its development, and/or (iv) relieve the infection, for example, eradication of the organism in infected subjects.

Passive immunization is accomplished by administering to a subject an effective amount of an anti-mannuronic acid antibody for inducing an immune response to alginate producing organisms, e.g. by causing opsonization of such organisms. "Passive immunity" as used herein involves the administration to a subject of antibodies produced in a different subject (including subjects of the same and different species), such that the antibodies cause the target organism (e.g., bacteria) to be phagocytosed.

The anti-mannuronic acid antibody may be administered to any subject at risk of developing an infection by an alginate producing organism. In some embodiments it may be particularly suited for subjects incapable of producing an active immune response to alginate or mannuronic acid. A subject incapable of producing an immune response includes an immunocompromised subject (e.g., patient undergoing chemotherapy, AIDS patient, etc.) and a subject that has not yet developed an immune system (e.g. pre-term neonate). Inability to form protective immune responses to alginate producing organisms has also been observed clinically, as discussed herein. These subjects will benefit from treatment with antibody preparations raised against mannuronic acid and its epitope, as described herein, because active vaccination may not be completely effective. Passive immunization therapy can also be used in subjects that already have an infection or related condition.

The anti-mannuronic acid antibody is administered to a subject in an effective amount for inducing a passive immune response. An "effective amount for inducing a passive immune response" as used herein is an amount of anti-mannuronic acid antibody that is sufficient to (i) prevent infection by alginate producing organisms from occurring in a subject which is exposed to such organisms, (ii) inhibit the development of infection, for example, arresting or slowing its development, and/or (iii) relieve the infection, for example, eradication of the organism in infected subjects. Using routine procedures known to those of ordinary skill in the art, one can determine whether an amount of antibody is an "effective amount for inducing a passive immune response" in an *in vitro* opsonophagocytic assay, as described herein. Preferably, the binding partner is an opsonic-killing antibody, and more preferably it is a human opsonic-killing antibody.

It is to be understood that the passive immunization methods of the invention do not intend to use the monoclonal antibodies F428, F429, F431 or Fcomb. Binding partners to be used in passive immunization may also exclude other mAb such as mAb M/K116.2, 8/5/31.3, and Ps 53, depending on the embodiment. Binding partners to be used in passive immunization exclude polyclonal sera, polyclonal antibody, and in some embodiments, non-human monoclonal antibodies (e.g., mouse monoclonal antibodies, rabbit monoclonal antibodies, rat monoclonal antibodies).

Preferably, the immune responses are cross-reactive across organisms that produce alginate.

Generally, a therapeutically effective amount may vary with the subject's age, condition, and sex, as well as the extent of the disease in the subject and can be determined by one of skill in the art. The dosage may be adjusted by the individual physician in the event of any complication.

An effective amount typically will vary from about 0.00001 mg/kg to about 1000 mg/kg, from about 0.0001 mg/kg to about 200 mg/kg, from about 0.0001 mg/kg to about 20 mg/kg, from about 0.0001 mg/kg to about 1 mg/kg, from about 0.0001 mg/kg to about 0.1 mg/kg, from about 0.0001 mg/kg to about 0.01 mg/kg, from about 0.0001 mg/kg to about 0.001 mg/kg in one or more dose administrations daily, for one or several days (depending of course of the mode of administration and the factors discussed above). Other suitable dose ranges include 1 µg to 200 µg per day, 1 µg to 150 µg per day, 1 µg to 100 µg per day, and 1 µg to 50 µg per day.

Single or multiple doses of the pharmaceutical compositions of the invention are contemplated. For example, active immunization schemes generally involve the administration of a high dose of an antigen followed by subsequent lower doses of antigen after a waiting period of several weeks. Further doses may be administered as well. The dosage schedule for passive immunization would be quite different with more frequent administration if necessary. Any regimen that results in an enhanced immune response to infection and/or subsequent protection from infection may be used. Desired time intervals for delivery of multiple doses of antigen can be determined by one of ordinary skill in the art employing no more than routine experimentation.

The antigens and/or binding partners (including binding peptides) may be delivered in conjunction with an anti-bacterial agent which includes antibiotics, bacterial antigens, or bacteria-specific antibodies. The compositions can therefore take the form of cocktails that include two, three or more of these components. In one embodiment, a common administration vehicle (e.g., tablet, implant, injectable solution, etc.) could contain both the composition of the invention and the antibiotic and/or antigen or antibody. Alternatively, the antibiotic and/or antigen or antibody can be separately dosed and administered from the compositions of the invention. The antibiotic can also be conjugated to the antigen or antibody.

Antibiotics are known in the art. They include but are not limited to penicillin G, penicillin V, ampicillin, amoxicillin, bacampicillin, cyclacillin, epicillin, hetacillin, pivampicillin, methicillin, nafcillin, oxacillin, cloxacillin, dicloxacillin, flucloxacillin, carbenicillin, ticarcillin, avlocillin, mezlocillin, piperacillin, amdinocillin, cephalexin, cephradine, cefadoxil, cefaclor, cefazolin, cefuroxime axetil, cefamandole, cefonicid, cefoxitin, cefotaxime, ceftizoxime, cefmenoxine, ceftriaxone, moxalactam, cefotetan, cefoperazone, ceftazidme, imipenem, clavulanate, timentin, sulbactam, neomycin, erythromycin, metronidazole, chloramphenicol, clindamycin, lincomycin, vancomycin, trimethoprim-sulfamethoxazole, aminoglycosides, quinolones, tetracyclines and rifampin. (See Goodman and Gilman's, Pharmacological Basics of Therapeutics, 8th Ed., 1993, McGraw Hill Inc.)

Polysaccharide antigens and antibodies are well known in the art. For instance, the following polysaccharide antigens and/or antibodies thereto can be administered in conjunction with the compositions of the invention: *Salmonella typhi* capsule Vi antigen (Szu et al. Infection and Immunity. 59:4555-4561 (1991)); *E. Coli* K5 capsule (Vann et al. European Journal of Biochemistry. 116: 359-364,(1981)); *Staphylococcus aureus* type 5 capsule (Fournier et al. Ann. Inst. Pasteur/Microbiol. (Paris). 138: 561-567, (1987)); *Rhizobium melilori* expolysaccharide II (Glazebrook et al. Cell. 65:661-672 (1989)); *Group B streptococcus* type III (Wessels et al. Journal of Biological Chemistry. 262:8262-8267 (1987)); *Pseudomonas aeruginosa* Fisher 7 O-specific side-chain (Knirel et al. European Journal of Biochemistry. 167:549, (1987)); *Shigella sonnei* O-specific side chain (Kenne et al. Carbohydrate Research. 78:119-126, (1980)); *S. pneumoniae* type I capsule (Lindberg et al. Carbohydrate Research. 78:111-117 (1980)); and *Streptococcus pneumoniae* group antigen (Jennings et al. Biochemistry. 19:4712-4719 (1980)).

The compositions may be administered substantially simultaneously with other therapies. By substantially simultaneously, it is meant that a composition of the invention is administered to a subject close enough in time with the administration of the other therapy (e.g., antibiotic administration) so that the two compounds may exert an additive or even synergistic effect.

The compositions, when used in vivo, are formulated as pharmaceutical preparations. In general, a pharmaceutical preparation is a preparation that comprises the antigen or binding partner of the invention in an effective amount, and optionally a pharmaceutically-acceptable carrier. As used herein, a pharmaceutically-acceptable carrier means a non-toxic material that does not interfere with the effectiveness of the biological activity of the active ingredients.

Pharmaceutically acceptable carriers include diluents, fillers, salts, buffers, stabilizers, solubilizers and other materials which are well-known in the art. Exemplary pharmaceutically acceptable carriers for peptides in particular are described in U.S. Patent No. 5,211,657. Such preparations may routinely contain salt, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

The compositions may be formulated into preparations in solid, semi-solid, liquid or gaseous forms such as tablets, capsules, powders, granules, ointments, solutions, depositories, inhalants and injections, and usual ways for oral, parenteral or surgical administration. The invention also describes pharmaceutical compositions which are formulated for local administration, such as by implants.

A variety of administration routes are available. The methods of the invention, generally speaking, may be practiced using any mode of administration that is medically acceptable, meaning any mode that produces effective levels of the active compounds without causing clinically unacceptable adverse effects. According to the methods of the invention the peptides can be administered by injection, by gradual infusion over time or by any other medically acceptable mode. Such modes of administration include oral, rectal, topical, nasal, interdermal, or parenteral routes. The term "parenteral" includes subcutaneous, intravenous, intramuscular, or infusion. Intravenous or intramuscular routes are not particularly suitable for long-term therapy and prophylaxis. They could, however, be preferred in emergency situations. Oral administration may be preferred for prophylactic treatment because of the convenience to the patient as well as the dosing schedule. Compositions suitable for oral administration may be presented as discrete units, such as capsules, tablets, lozenges, each containing a predetermined amount of the active agent. Other compositions include suspensions in aqueous liquids or non-aqueous liquids such as a syrup, elixir or an emulsion.

When the compounds described herein (including peptide and non-peptide varieties) are used therapeutically, in certain embodiments a desirable route of administration may be by pulmonary aerosol. Techniques for preparing aerosol delivery systems containing compounds are well known to those of skill in the art. Generally, such systems should utilize components which will not significantly impair the biological properties of the peptides (see, for example, Sciarra and Cutie, "Aerosols," in Remington's Pharmaceutical Sciences, 18th edition, 1990, pp 1694-1712; incorporated by reference). Those of skill in the art can readily determine the various parameters and conditions for producing aerosols without resort to undue experimentation.

The compositions of the invention may be administered directly to a tissue. Preferably, the tissue is one in which the infection exists, such as for example, the lungs in cystic fibrosis patients. Alternatively, the tissue is one in which the infection is likely to arise. Direct tissue administration may be achieved by direct injection. The compositions may be administered once, or alternatively they may be administered in a plurality of administrations. If administered multiple times, the compositions may be administered via different routes. For example, the first (or the first few) administrations may be made directly into the affected tissue while later administrations may be systemic.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Lower doses will result from other forms of administration, such as intravenous administration. In the event that a response in a subject is insufficient at the initial doses applied, higher doses (or effectively higher doses by a different, more localized delivery route) may be employed to the extent that patient tolerance permits. Multiple doses per day are contemplated to achieve appropriate systemic levels of compounds.

Other delivery systems can include time-release, delayed release or sustained release delivery systems. Such systems can avoid repeated administrations of the agent, increasing convenience to the subject and the physician. Many types of release delivery systems are available and known to those of ordinary skill in the art. They include polymer base systems and non-polymer systems such as lipids including sterols such as cholesterol, cholesterol esters and fatty acids or neutral fats such as mono- di- and tri-glycerides; hydrogel release systems; silastic systems; peptide based systems; wax coatings; compressed tablets using conventional binders and excipients; partially fused implants; and the like. Specific examples include, but are not limited to: (a) erosional systems in which the agent is contained in a form within a matrix such as those described in U.S. Patent Nos. 4,452,775, 4,675,189, and 5,736,152 and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Use of a long-term sustained release implant may be particularly suitable for prophylactic treatment of subjects at risk of developing an infection. Long-term release, as used herein, means that the implant is constructed and arranged to delivery therapeutic levels of the active ingredient for at least 30 days, and preferably 60 days. Long-term sustained release implants are well-known to those of ordinary skill in the art and include some of the release systems described above.

The delivery vehicle is a biocompatible microparticle or implant that is suitable for implantation into the mammalian recipient. Exemplary biodegradable implants that are useful in accordance with this method are described in PCT International Application No. PCT/US/03307 (Publication No. WO 95/24929, entitled "Polymeric Gene Delivery System", claiming priority to U.S. patent application serial no. 213,668, filed March 15, 1994). PCT/US/0307 describes a biocompatible, preferably biodegradable polymeric matrix for containing a biological macromolecule. The polymeric matrix may be used to achieve sustained release of the agent in a subject. The agent described herein may be encapsulated or dispersed within the biocompatible, preferably biodegradable polymeric matrix disclosed in PCT/US/03307. The polymeric matrix preferably is in the form of a microparticle such as a microsphere (wherein the agent is dispersed throughout a solid polymeric matrix) or a microcapsule (wherein the agent is stored in the core of a polymeric shell). Other forms of the polymeric matrix for containing the agent include films, coatings, gels, implants, and stents. The size and composition of the polymeric matrix device is selected to result in favorable release kinetics in the tissue into which the matrix device is implanted. The size of the polymeric matrix device further is selected according to the method of delivery which is to be used, typically injection into a tissue or administration of a suspension by aerosol into the nasal and/or pulmonary areas. The polymeric matrix composition can be selected to have both favorable degradation rates and also to be formed of a material which is bioadhesive, to further increase the effectiveness of transfer when the device is administered to a vascular, pulmonary, or other surface. The matrix composition also can be selected not to degrade, but rather, to release by diffusion over an extended period of time.

Both non-biodegradable and biodegradable polymeric matrices can be used to deliver the agents of the invention to the subject. Biodegradable matrices are preferred. Such polymers may be natural or synthetic polymers. Synthetic polymers are preferred. The polymer is selected based on the period of time over which release is desired, generally in the order of a few hours to a year or longer. Typically, release over a period ranging from between a few hours and three to twelve months is most desirable. The polymer optionally is in the form of a hydrogel that can absorb up to about 90% of its weight in water and further, optionally is cross-linked with multivalent ions or other polymers.

In general, the agents may be delivered using the biodegradable implant by way of diffusion, or more preferably, by degradation of the polymeric matrix. Exemplary synthetic polymers which can be used to form the biodegradable delivery system include: polyamides, polycarbonates, polyalkylenes, polyalkylene glycols, polyalkylene oxides, polyalkylene terepthalates, polyvinyl alcohols, polyvinyl ethers, polyvinyl esters, poly-vinyl halides, polyvinylpyrrolidone, polyglycolides, polysiloxanes, polyurethanes and co-polymers thereof, alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, poly vinyl chloride, polystyrene, polymers of lactic acid and glycolic acid, polyanhydrides, poly(ortho)esters, polyurethanes, poly(butic acid), poly(valeric acid), and poly(lactide-cocaprolactone) and polyvinylpyrrolidone.

Examples of biodegradable polymers include natural polymers such as alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water *in vivo,* by surface or bulk erosion.

Examples of non-biodegradable polymers include ethylene vinyl acetate, poly(meth)acrylic acid, polyamides, copolymers and mixtures thereof.

Bioadhesive polymers of particular interest include bioerodible hydrogels described by H.S. Sawhney, C.P. Pathak and J.A. Hubell in Macromolecules, 1993, 26, 581-587, the teachings of which are incorporated herein, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

The following examples are provided to illustrate specific instances of the practice of the present invention and are not intended to limit the scope of the invention. As will be apparent to one of ordinary skill in the art, the present invention will find application in a variety of compositions and methods.

### Examples

### Materials and Methods

*Bacterial strains.* PAO1 (sensitive to chloramphenicol) and PA14 were provided by Dr. Michael Vasil, Denver, CO and Dr. Fredrick Ausubel, Boston, MA respectively. Production of strain PAO1 expressing the ExoU cytotoxin was previously described (19). PAO1 and PA14 strains unable to produce alginate due to in-frame interruptions in the algD gene essential for alginate synthesis (ΔalgD strains) were provided by Drs. Dennis Ohman and Sang Jin Suh, Richmond, VA (24) and Dr. Peter Yorgey, Boston, MA (25), respectively. Clinical isolates of mucoid *P. aeruginosa* from CF patients were obtained from microbiology laboratories around the United States. Clinical isolates of non-mucoid *P. aeruginosa* from CF patients early in the course of the disease were obtained from Children's Hospital, Boston or provided by Dr. Jane Burns, Children's Hospital, Seattle (3). Clinical isolates from patients with *P. aeruginosa* bacteremia were provided by Dr. Reuben Ramphal, Gainesville, Fl.

*Production of mAbs.* A single volunteer was immunized subcutaneously with 100 µg of a previously described preparation of purified alginate (referred to as MEP, (13)). Seven days later, peripheral blood mononuclear cells (PBMC) were isolated from 50 ml of blood using Ficoll Hypaque sedimentation and B cells stimulated by overnight exposure to Epstein-Barr virus (EBV) produced from the B95.8 cell line as described (26). After 24 hours, the cells were washed and dispersed into 96 well plates at a concentration of 1x10⁶ PBMC/well in 100 µl of growth media (RPMI1640 supplemented with 20% FBS) containing 10% Lymphocyte Conditioned Medium (LyCM, prepared from human PBMC stimulated for 48 hours with phytohemagglutinin). After 5 days, an additional 100 µl of growth media supplemented with 10% LyCM was added. EBV stimulated cells were then fed weekly by removal of 100 µl of spent media and the addition of 100 µl of growth media supplemented with 10% LyCM. When the wells were densely seeded as evidenced by growth over 80% of the bottom of the well and the appearance of a pH change in the media indicative of cellular growth, the cultures were screened for production of specific antibody to *P. aeruginosa* alginate by ELISA as described below.

The cells from single individual wells giving a positive reaction for antibody were then dispersed into 48 wells of a tissue culture plate and after several days of growth the supemates tested for reactivity with specific alginate antigen. Cultures that continued to test positive were then fused with a human-mouse myeloma cell line, HMMA 2.5 to generate hybridomas as previously described (27). After fusion, cells were cultured in microwell plates with growth medium (RPMI 1640 supplemented with 20% FBS and hypoxanthine-aminopterin-thymidine and oubain) for selection of fused cells. These cultures were fed at weekly intervals and screened by ELISA for antibody production. Hybridomas were cloned at a density of 1 cell/well, wells with positive growth screened by ELISA for specific antibody, and wells containing positive antibody-producing hybridomas expanded into wells in tissue culture plates of increasing volume then flasks of increasing volume to obtain cloned cell lines. Three hybridomas, designated F428, F429 and F431, producing human IgA antibodies with lambda light chains positive for binding to alginate were chosen for further study.

*Production of recombinant IgG human mAbs.* Heavy and light chain variable region genes were cloned from the 3 IgA-producing hybridomas using PCR. mRNA was isolated from the hybridomas, reverse transcribed to cDNA and then PCR amplification carried out as described (28) using forward primers for 6 conserved leader sequences of the 5' end of the human IGHV genes (Table I) along with a single 3' reverse primer complementary to the beginning of the 5' end of the IGHA gene (Table I). Similarly, PCR amplification of the 1GLV gene was accomplished using 7 forward primers for the 5' end of conserved leader sequences for the human IGLV genes along with a single 3' reverse primer complementary to the 5' end of the human IGLC gene (Table I). The primers were designed to incorporate restriction sites for insertion into the TCAE 6.2 vector (29) as described previously for insertion of PCR products into the closely related TCAE 5.3 vector (28). PCR products were cloned into the pCR2.1 vector, transformed into E. *coli* as described (28) and plasmids isolated from 3-5 clones of each PCR product, which were then analyzed by restriction enzyme analysis to indicate that a particular clone had inserted DNA of the expected size. Three to 5 clones of each IGHV-D-J and IGLV-J gene were then chosen for sequence analysis. Results indicated the cloning of 2 distinct IGHV-D-J and 2 distinct IGLV-J genes from hybridomas F428, F429 and F431. One of the clones from the group giving 3 of 3 or 4 of 5 identical sequences for each IGHV-D-J and IGLV-J product was used to insert into the TCAE 6.2 vector for production of 4 recombinant human IgG1 antibodies, representing all combinations of the 2 IGHV-D-J and 2 IGLV-J genes. These included the original 3 combinations of IGHV-D-J and IGLV-J in hybridomas F428, F429 and F431 and the fourth possible combination in a clone designated FCOMB. Analysis of the germ-line gene usage of the cloned IGHV, IGHD, IGHJ, IGLV and IGLJ genes was carried out using IMGT/V QUEST (6).

*Creation of recombinant human IgG1 antibody.* Vector TCAE 6.2 is similar to the previously described vector TCAE 5.3 (29) except TCAE 6.2 contains the genomic sequence of the human IGLC constant region in place of the human IGKC constant region. Plasmids containing the 2 entire IGHV-D-J sequences were digested with Nhe1 and Mlu, the desired fragment isolated by gel electrophoresis and ligated into TCAE 6.2 digested with the same restriction enzymes for in-frame insertion 5' to the human IGHG1 constant region gene. After electroporation into E. coli, plasmids were recovered and the inserted DNA checked by sequence analysis of a PCR product to insure the proper DNA sequence was contained in the vector. Next, plasmids containing the 2 entire IGLV-J clones were digested with BsiWI and DraIII, and the desired DNA fragments were recovered by gel electrophoresis and ligated into similarly digested TCAE 6.2 plasmids to yield a gene in-frame with the human IGLC constant region gene that already contained one of the 2 IGHV-D-J genes obtained from the original 3 hybridomas. The constructed plasmids were electroporated into *E. coli,* recombinant plasmids isolated, and again the inserted DNA was checked by sequence analysis to verify that the desired IGLV-J DNA sequences had been properly introduced.

*Production of recombinant human IgG1 antibody.* The 4 constructed plasmids were transfected into the DG44 line of Chinese Hamster Ovary (CHO) cells using DNA-liposome mediated transfection as described (28). DG44 lacks both chromosomal copies of the dihydrofolate reductase (dhfr) gene while vector TCAE 6.2 contains this gene, allowing for selection and amplification of cells containing transfected DNA in the presence of methotrexate, which is detoxified by the dhfr gene. Cells were initially grown in F12 medium with 10% FBS and 400 µg G418/ml and clones established by limiting dilution plating in 96-well tissue culture plates. Wells with clonal growth were checked for the production of the recombinant IgG1 antibody as described (28), using ELISA plates coated with anti-human IgG as a capture antibody, supernates from DG44 clones. Detection was performed with an anti-human IGLC-specific secondary antibody. The clones positive for the greatest reactivity of the recombinant antibody were then screened for binding to alginate isolated from *P*. *aeruginosa* mucoid strain 2192 as described (20). Selected clones producing high levels of antigen-specific antibody were grown up in progressively larger volumes. Once stable antibody-producing clones were established, the cells were grown in progressively decreasing concentrations of FBS until they were replicating well in serum free medium.

To amplify the dhfr and associated genes for antibody production, clones were replated in 96-well tissue culture plates in the presence of 5 nM methotrexate. Cultures were scaled up into T75 flasks, supernate screened for antibody production, and further amplification of dhfr and Ig sequences carried out by increasing the concentration of methotrexate to 50 nM, and then to 500 nM in serum-free F12 medium. Recombinant antibody was isolated from bulk cultures grown either in tissue culture flasks or a 2 L spinner flask contained in a 5% CO₂ incubator by running the clarified (by centrifugation) cell-free supernate directly onto a Protein-G column and eluting the bound antibody with 0.1 M glycine buffer, pH 2.7.

*Production of recombinant IgG2, IgG3 and IgG4 antibody.* Exchange of the IGHG1 gene in the recombinant plasmid containing the combination of IGHV-D-J and IGLV-J genes originally found in hybridoma F429 for heavy chain constant region genes IGHG2, IGHG3 or IGHG4, was carried out as previously described (28). Transfection of DG44 cells with these constructs was also as previously described and summarized above (28), as was production of recombinant IgG2, IgG3 and IgG4 antibody.

*Serologic analyses.* ELISA assays using purified or reduced *P. aeruginosa* alginate or polymannuronic acid as coating antigens and all subsequent steps, were carried out as previously described (18, 20). Similarly, phagocyte-dependent killing assays were carried out as described (11, 20) using the recombinant mAbs as antibody sources and infant rabbit serum as a complement source. Although statistically significant (P<.05) killing at a level of a 20-40% reduction in surviving cfu can be obtained, reductions of <40% are not considered of biologic significance as they are not associated with protective efficacy, so all determinations of a significant killing activity were based on both a statistical analysis using a t-test comparing tubes with human mAb to alginate with tubes containing a control IgG1 myeloma (Sigma, St. Louis, MO).

*Protection against non-mucoid P. aeruginosa in an acute murine lung infection model.* Mice were either 6-8 week old C3H/HeN or C57/B16 females. In order to deliver high quantities of the recombinant human mAbs to the murine lung, mice were anesthetized by IP injection of ketamine (20- 30 mg/Kg) and xylazine (5 mg/Kg) and 10-50 µl of a 1 mg/ml concentration of either the IgG1 mAb to alginate or a control human IgG1 myeloma (Sigma) mAb placed on the nares until 10 to 50 µg of antibody were instilled. This dose was initially given as a single bolus 4 hours prior to infection. In subsequent experiments, 50 µg was delivered at either 48, 24 or 4 hours prior to infection in various combinations to deliver total doses of 50-150 µg recombinant or control antibody/mouse. Intranasal infection of anesthetized mice to produce an acute pneumonia using lethal concentrations of different *P. aeruginosa* strains contained in 20 µl was as described (19, 30). Mice were followed until moribund, when they were sacrificed, or dead, and all such outcomes were counted as lethal events.

*Promotion of pulmonary clearance of mucoid P. aeruginosa.* As most mucoid isolates of *P. aeruginosa* do not cause a lethal lung infection except at very high inocula, a clearance model was used to evaluate the recombinant human mAb for in vivo activity against these strains. Intranasal delivery of 100 µg of recombinant or control IgG1 mAb in two 50 µg doses 24 and 4 hours prior to infection was as described above. Intranasal infection with the mucoid strains was also as described above. Infected mice were sacrificed at 2, 4 or 24 hours post-infection, both lungs removed, weighed, homogenized and the homogenate diluted and plated for determinations of cfu/gm lung tissue as described (19, 30).

*Statistical analysis.* P values for the significance of the differences in survival in the acute pneumonia model were calculated using Fisher's exact test. P values for the differences in cfu/gm lung tissue in the pulmonary clearance model were determined by a t-test.

### Results

*Characteristics of the initial hybridomas and their variable region genes.* 3 IgA secreting hybridomas designated F428, F429 and F431 were obtained that bound to purified alginate in an ELISA. The complete heavy and light chain antibody V regions were cloned and sequenced from cDNA prepared from mRNA being produced by these 3 hybridomas. Analysis of the nucleotide and amino acid sequences indicated that clones F428 and F429 contained the same H chain V region but different L chain V regions, whereas clone F431 had a distinct H chain V region but shared the L chain V region of clone F428 (GenBank accession numbers: AY62666: IGLV-J of mAbs F428 & F431; AY626662: IGLV-J of mAb F429; AY626663: IGHV-D-J of mAbs F428 & F429; AY626664: IGHV-D-J of mAb F431). The germ line IGHV, IGHD, IGHJ, IGLV and IGLJ genes used to produce these two different H and L chains were determined by sequence alignment analysis (Table II). The cloned V regions were then inserted in the TCAE 6.2 vector to produce 4 different human IgG1 antibody clones, representing the 3 original clones and a fourth clone, designated Fcomb, which expressed the H chain V region of mAb F431 and L chain V region of mAb F429.

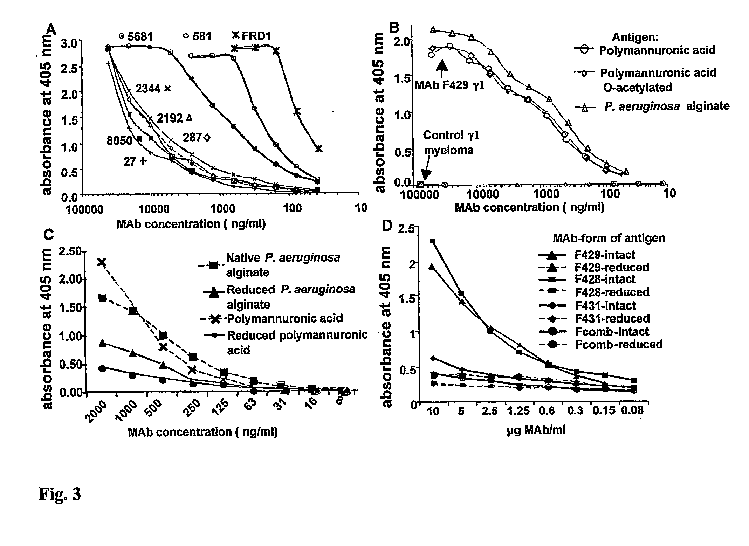

*Antigen binding characteristics.* All 4 of the purified human IgG1 mAbs had comparable binding to alginate isolated from a single mucoid strain (2192) of *P. aeruginosa* (not shown). However, after further analysis it was found that specific IGHV and IGLV regions in mAb F429 had broader overall antigen-binding activity when this particular MAb was screened using alginate isolated from 8 different mucoid strains (Fig. 3A).

Analysis of the epitopic specificity of mAb F429 as an IgG1 antibody showed no difference in mAb binding to native or deacetylated alginate from *P. aeruginosa* strain 2192 (not shown), a finding distinct from prior results with polyclonal antibody raised to an alginate-KLH conjugate vaccine (20). To determine if there was another epitopic specificity for this mAb, purified polymannuronic acid was used in an ELISA and strong binding of mAb F429 to this antigen was observed, which was not further increased by introduction of acetate groups (Fig. 3B). A control human IgG1 myeloma did not bind to any of the tested antigens. To determine if the carboxyl groups on the C6 carbon forming the uronic acid moiety were involved in mAb binding, both native alginate from *P. aeruginosa* and polymannuronic acid were treated with water soluble carbodiimide to reduce the carboxyl groups, and this resulted in decreased binding of mAb F429 to both antigens (Fig. 3C). When the other 3 mAbs were tested with intact and reduced polymannuronic acid, mAb F428, which shares the H chain with mAb F429, had the same pattern of reactivity while mAbs F431 and Fcomb did not bind well to either intact or reduced polymannuronic acid (Fig. 3D). Thus, mAbs F428 and F429 showed antigenic specificity for an epitope comprising intact carboxyl groups on the C6 carbon within the blocks of mannuronic acids found in *P. aeruginosa* alginate.

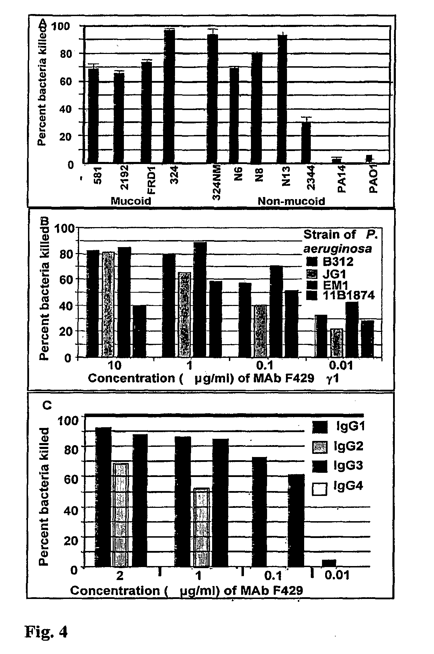

*Phagocytic killing activity.* All 4 of the mAbs showed good phagocytic killing against a panel of mucoid strains (not shown) but mAb F429γ1 had the best overall activity (Fig. 4A) and mAb F428γ1 had comparable activity (not shown). Lower concentrations of mAb F429γ1 than those shown in Fig. 4 had appropriately decreasing killing activity (not shown). Surprisingly, when tested against a panel of non-mucoid strains isolated from CF patients early in the course of infection, there was excellent killing of these strains by mAb F429γ1 (Fig. 4A). mAbs F431 and Fcomb, which bound poorly to polymannuronic acid, were essentially unable to kill non-mucoid *P. aeruginosa* strains (not shown). When mAb F429 was tested against *P. aeruginosa* strains PA14 and PAO1, known to express low levels of alginate in vitro (31), there was very little phagocytic killing.

To determine if non-mucoid clinical isolates of *P. aeruginosa* from another tissue were similarly susceptible to killing by mAb F429γ1, 4 *P. aeruginosa* isolates from blood were tested. All were opsonized to a high level by mAb F429γ1 (Fig. 4B).

The IGHG1 gene for mAb F429γ1 was changed to IGHG2, IGHG3 and IGHG4 to produce human IgG2, IgG3 and IgG4 mAbs with the same IGHV-D-J and IGLV-J genes. Determination of the binding of the mAbs of these 4 isotypes to *P. aeruginosa* alginate by ELISA using an IGLC-specific secondary antibody showed essentially identical binding of all 4 mAbs, indicating no effect of the Fc-component of the IGHC region on antigen binding. When the mAbs were evaluated for killing of mucoid *P. aeruginosa* strain 324, IgG1 and IgG3 mAbs mediated the best killing, while the IgG2 mAb had less activity and the IgG4 mAb was virtually without killing activity (Fig 4C). As phagocyte-dependent killing of P. *aeruginosa* by antibody to alginate is completely complement dependent (10), the differences in phagocytic killing likely relate to the complement-activating properties of the mAbs of the different IgG isotypes.

*Protection against acute pneumonia by non-mucoid clinical isolates of P. aeruginosa.* As mAb F429 mediated killing of non-mucoid, LPS smooth isolates of *P. aeruginosa* that are virulent in an acute lung infection model (19) whether the mAb was also protective against in vivo infection was initially evaluated. Preliminary studies showed that in order to achieve sufficient levels of the mAb within the lung early in infection it had to be delivered intranasally, while intraperitoneal delivery of the mAbs did not result in sufficient levels in the infected lungs within 3 hours of bacterial inoculation to exert an appreciable decline in microbial numbers (not shown). The protective efficacy of a single dose of mAb F429γ1 was evaluated against challenge with strain N13, an early non mucoid clinical isolate from a CF patient. As shown in Table III, doses of 100 and 50 µg/mouse given 4 hours prior to infection provided complete protection against infection when compared with mice given an irrelevant human IgG1 myeloma. High-level protection was also achieved with mAb F428γ1, which shares the same H chain and antigenic specificity for intact but not reduced polymannuronic acid with mAb F429γ1. Doses of 25 µg mAb F429γ1/mouse (Table III) or less (not shown) were without efficacy. 100 µg of mAbs F431 and Fcomb, which do not bind well to the polymannuronic acid antigen, were not protective.

The poor killing of two *P. aeruginosa* strains, PAO1 and PA14, suggested mAb F429γ1 would not be protective against challenge with these two strains, but in fact there was significant protection against lethal pneumonia due to both strains (Table III). Strain PAO1 is not very virulent in C3H/HeN mice, so the protection study was repeated in C57B1/6 mice, which are more susceptible to acute *P. aeruginosa* pneumonia, requiring a lower dose for full lethality in controls. Complete protection against challenge of C57B1/6 mice with a lethal dose of PAO1 was achieved by 100 µg of mAb F429γ1.

The specificity of the mAb for alginate was shown by testing its protective efficacy against strains PAO1ΔalgD and PA14ΔalgD which cannot produce alginate. No protection was seen with these strains (Table III), which were tested in the same experiment with the parental strains for which full protection was achieved.

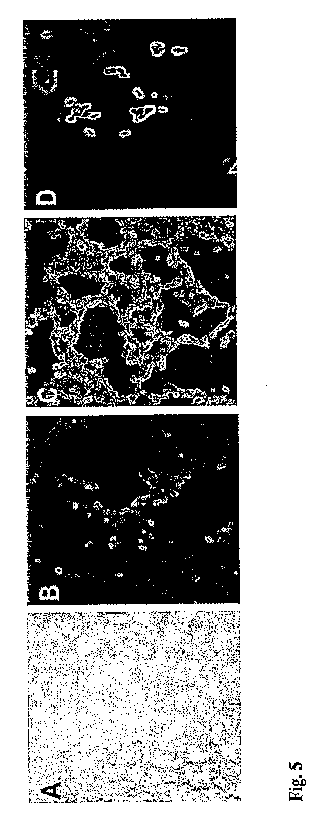

Evaluation of in vivo alginate expression in the lungs of mice immediately after inoculation of strain PAO1 showed no detectable alginate (Fig. 5) (20). However, by one hour post-infection *P. aeruginosa* bacteria strongly expressing alginate in vivo were readily observed in lung sections reacted with alginate-specific antibodies (Fig. 5). A similar finding of alginate expression 24 hours post-infection in the lungs of mice infected with strain PAO1 embedded in agar beads has been found (A. Bragonzi et al., submitted for publication). Thus, within 1 hour of inoculation, *P. aeruginosa* alginate appears to be rapidly produced by strain PAO1, and this likely accounts for the in vivo protective efficacy of mAb F429γ1 against this strain, and possibly against strain PA14.

Testing the protective efficacy of mAb F429γ1 against 4 additional non-mucoid P. *aeruginosa* strains confirmed that this mAb could provide complete protection against lethal doses of non-mucoid strains (Table III), including strain PAO1 ExoU⁺, a recombinant strain expressing the ExoU cytotoxin that increases the disease morbidity and mortality of *P. aeruginosa* in both animals (19) and humans (32, 33).

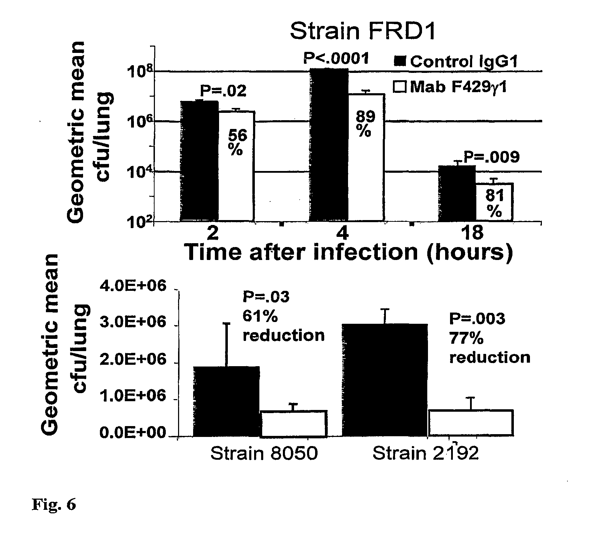

*Enhanced clearance of mucoid P. aeruginosa from murine lungs.* Most mucoid *P. aeruginosa* strains do not cause lethal infections in mice except at very high doses, due to the fact they are LPS rough strains with virtually no ability to disseminate systemically. Additionally, other models of mucoid *P. aeruginosa* infection such as embedding the organisms in agar or alginate beads are limited due to the poor ability of most mucoid strains to establish infections in non-immune mice. In order to show that mAb F429 recognized an epitope expressed in vivo by mucoid strains of *P. aeruginosa,* the ability of this mAb to promote clearance from the lungs of mice of 3 different clinical isolates of mucoid *P*. *aeruginosa* was evaluated. Mice given 100 µg of mAb F429γ1 IN in two 50 µg doses 24 and 4 hours prior to IN infection had significantly enhanced clearance of mucoid strain FRD1 after 2, 4 and 18 hours of infection compared with mice given a control IgG1 myeloma (Fig. 6). Similarly, at 4 hours post-infection, mAb F429γ1 significantly enhanced clearance of mucoid strains 2192 and 8050 (Fig. 6). Thus, the epitope recognized on alginate by mAb F429 was available on mucoid strains in the lungs of mice for effective targeting for immune elimination.

**Table I. Sequences of primers used to clone variable regions of mAbs to P. aeruginosa alginate.**

| Heavy chain V region, 5' forward primers: | | |
|---|---|---|
| VH1LDRHU | CCATGGACTGGACCTGGA | (SEQ ID NO:1) |
| VH2LDRHU | ATGGACATACTTTGTTCCAC | (SEQ IQ NO:2) |
| VH3LDRHU | CCATGGAGYYKGGGCTGAGC | (SEQ ID NO:3) |
| VH4LDRHU | ATGAACAYCTGTGGTTCTT | (SEQ ID NO:4) |
| VH5LDRHU | ATGGGGTCAACCGCCATCCT | (SEQ ID NO:5) |
| VH6LDRHU | ATGTCTGTCTCCTTCCTCAT | (SEQ ID NO:6) |

| Heavy chain N terminus of IgA constant region, 3' reverse primer: | | |
|---|---|---|
| | CAGCGGGAAGACCTTGG | (SEQ IQ NO:7) |

| Lambda light chain V region, 5' forward primers: | | |
|---|---|---|
| lambda sig1 | AGATCTCTCACCATGGCCRGCTTCCCTCTCCTC | (SEQ ID NO:8) |
| lambda sig2 | AGATCTCTCACCATGACCTGCTTCCCTCTCCTC | (SEQ ID NO:9) |
| lambda sig3 | AGATCTCTCACCATGGCCTGGGCTCTGCT | (SEQ ID NO:10) |
| lambda sig4 | AGATCTCTCACCATGACTTGGATCCCTCTCTTC | (SEQ ID NO:11) |
| lambda sig5 | AGATCTCTCACCATGGCATGGATCCCTCTCTTC | (SEQ ID NO:12) |
| lambda sig6 | AGATCTCTCACCATGGCCTGGACCCCTCTCTGG | (SEQ ID NO:13) |
| lambda sig7 | AGATCTCTCACCATGGCCTGGATGATGCTTCTC | (SEQ ID NO:14) |

| Light chain, N terminus of lambda chain constant region, 3' reverse primer: | | |
|---|---|---|
| | GGAGGGCGGGAACAGAGTGAC | (SEQ ID NO:15) |

**Table II. Germline gene usage of 3 IgA hybridomas secreting mAbs to P. aeruginosa alginate.**

| Hybridoma | Germline genes used for entire V region | | |
|---|---|---|---|
| H or L chain | Variable region | Diversity region | Joining region |
| F428H | IGHV4-39 | IGHD3-10 | IGHJ4 |
| F428L | IGLV1-51 | | IGLJ1 |
| F429H | IGHV4-39 | IGHD3-10 | IGHJ4 |
| F429L | IGLV1-51 | | IGLJ3 |
| F431H | IGHV4-39 | IGHD6-25 | IGHJ5 |
| F431L | IGLV1-51 | | IGLJ1 |

**Table III. Protection against lethal pneumonia in mice by mAbs to P. aeruginosa alginate.**

| Challenge dose (CFU/mouse) Mouse strain | Challenge strain of *P. aeruginosa* (source) | mAb given | mAb dose (µg) per mouse | Survivors/total: Controls¹ | Survivors/ total: Mab | P value² |
|---|---|---|---|---|---|---|
| 5 X 10⁷ | N13 (CF) | F429γ1 | 100 | 0/6 | 6/6 | .001 |
| C3H/HeN | | | 50 | 0/6 | 6/6 | .001 |
| | | | 25 | 0/6 | 0/6 | NS³ |
| | | F428γ1 | 100 | 0/10 | 8/10 | <.0001 |
| | | F431γ1 | 100 | 0/6 | 0/6 | NS |
| | | Fcombγ1 | 100 | 0/6 | 0/6 | NS |
| 2 X 10⁸ | PAO1 (Lab) | F429γ1 | 150 | 0/6 | 4/6 | .03 |
| C3H/HeN | PAO1Δ*alg*D | | | 0/6 | 0/6 | NS |
| 2 X 10⁷ | PAO1 (Lab) | F429γ1 | 100 | 0/5 | 5/5 | .002 |
| C57Bl/6 | PAO1Δ*alg*D | | | 0/6 | 0/6 | NS |
| 3 X 10⁸ | PA14 (Burn) | F429γ1 | 100 | 0/6 | 5/5 | .002 |
| 4 X 10⁷ | PA14Δ*alg*D | | | 0/6 | 0/6 | NS |
| C3H/HeN | | | | | | |
| 2 X 10⁷ | PAO1 ExoU⁺ | F429γ1 | 100 | 0/5 | 6/6 | .002 |
| C3H/HeN | (Lab) | | | | | |
| 4 X 10⁷ | N6 (CF) | F429γ1 | 100 | 0/6 | 6/6 | .001 |
| C3H/HeN | | | | | | |
| 6 X 10⁷ | B312 | F429γ1 | 150 | 0/6 | 6/6 | .001 |
| C3H/HeN | (Blood) | | | | | |
| 6 X 10⁷ | 1B1874-2 | F429γ1 | 100 | 0/6 | 6/6 | .001 |
| C57Bl/6 | (Blood) | | | | | |

| | | | | | | |
|---|---|---|---|---|---|---|
| ¹Controls given an equal amount of a human IgG1 myeloma ² P value determined by Fisher's exact test ³ NS, not significant (P > .05) | | | | | | |

### Discussion

Modem molecular genetic techniques are now sufficiently developed to produce high levels of fully human mAbs for evaluation of important immunologic functions. One area of application of this technology is production of protective human mAbs to microbial antigens such as polysaccharides that are often poorly immunogenic in target populations, may have immunodominant epitopes that are not targets for protective antibody such as occurs in HIV infection, or may induce a non-protective antibody isotype. In the case of *P. aeruginosa,* the alginate antigen produced by virtually all strains induces natural antibody in most humans (7), as well as high titers of antibody in chronically infected CF patients (7), but these antibodies fail to promote phagocyte-dependent bacterial killing or provide protection against infection. In addition, immunization of humans with purified alginate failed to induce killing antibody in most vaccinates (14). It has been shown in accordance with the invention that non-protective IgA antibodies made in response to immunization of a human volunteer with alginate could be engineered into IgG antibodies that mediated killing of both mucoid and non-mucoid *P. aeruginosa* strains and protected against lung infections with these strains.

Passive therapy of *P*. *aeruginosa* infections with an appropriate mAb would be useful for prophylactic and therapeutic interventions in patients at risk for or having acquired infection. These include CF patients early in life before serious infection has set in (34, 35), patients undergoing chemotherapy that increases the risk for *P. aeruginosa* infection (36, 37), burn patients (38) and patients on respirators where *P. aeruginosa* ventilator-associated pneumonia remains a major problem (33, 39). Although it was found that topical delivery of mAb F429γ1 to the lungs of mice gave maximal efficacy in protecting against lethal infection with non-mucoid *P. aeruginosa* strains or promoting clearance of mucoid *P. aeruginosa* strains, it is likely that systemic delivery of this or similar mAbs to humans would be efficacious. Bacterial challenge doses in the murine models are high in order to induce a significant pathologic effect, whereas actual infecting doses in humans are likely much lower and thus more amenable to control with the lower amounts of systemically-delivered mAbs derived from the circulation. The humanized mAb Palivizumab that is used clinically for prophylaxis against respiratory syncytial virus infection is given at a dose of 15 mg/Kg (40), which translates into a dose of about 375-450 µg of mAb to a 25-30 g mouse. Thus the use of 50-150 µg delivered topically to the lung is within the range of mAb doses used in human clinical medicine.

Several *P. aeruginosa*-specific hyperimmune intravenous IgG (IVIg) passive therapeutic reagents have been tried in various patient populations over the years. Efficacy against burn wound infection using immune globulin from patients immunized with a cellular extract of *P. aeruginosa* showed protective efficacy in the early 1980s (41, 42) but this product was not further pursued. A hyperimmune IVIg product prepared from plasma donors immunized with an octavalent *P. aeruginosa* LPS O-side chain conjugate vaccine (43, 44) showed no protective efficacy in patients in intensive care units (45). This may have been due to low antibody coverage for a sufficient diversity of serologically distinct O antigens, or possibly to an overall low infection rate precluding an adequate sample size for documentation of the IVIg's efficacy.

The demonstration here that a fully human mAb to *P. aeruginosa* alginate mediates phagocyte-dependent killing and is protective against both non-mucoid and mucoid strains of *P. aeruginosa* was unexpected. Although many non-mucoid strains have been shown to produce low levels of alginate in vitro (17, 18), others do not (31) and it was not clear if the level of alginate expression by non-mucoid strains was sufficient for it to serve as a target for killing and protective antibody. Prior results with polyclonal rabbit antibodies to alginate conjugates indicated non-mucoid strains were poorly killed by such antibodies (20). Similarly, here it was found that strains PAO1 and PA14, which produce little to no detectable alginate in vitro, were poorly killed by mAb F429, but nonetheless it was possible to protect against infection with these isolates in the murine acute pneumonia model. This was shown to be due to an increase by 1 hour post-infection in alginate expression in vivo by strain PAO1, and presumably the same explanation is operative with strain PA14. Specificity of mAb F429 for alginate was shown by its inability to protect against infection with *alg*D deleted strains of PAO1 and PA14. For the other non-mucoid clinical isolates, sufficient alginate was expressed in vitro for killing by mAb F429 to proceed, and protection against acute pneumonia was achieved. Thus, mAb F429 appears to have high potential utility for protecting against non-mucoid strains typical of nosocomial isolates of *P*. *aeruginosa.*

Mucoid strains also expressed the binding epitope of mAb F429 in a proper conformation and density for the antibody to mediate killing of these strains and promote their clearance from mouse lungs. Previously characterized polyclonal antisera raised to purified alginate, alginate conjugates or murine mAbs to alginate appeared to recognize epitopes formed by O-acetyl substituents on the C2 and C3 hydroxyl groups of the mannuronic acid residues (11, 15, 20, 46). However, the protective mAb F429 did not have this antigenic specificity, binding equally well to acetylated and deacetylated alginate. Further investigations showed the mAb bound to blocks of mannuronic acid regardless of the presence of O-acetate substituents, but dependent on an intact carboxyl group on C6 that forms the uronic acid moiety. This specificity appears critical for the killing and protective activity of mAbs F428 and F429 against non-mucoid strains, as these strains are not opsonized by polyclonal antisera raised to an alginate conjugate vaccine whose epitopic specificity is towards acetylated alginate (20).

The apparent broad protective efficacy of mAb F429 against both mucoid and non mucoid strains of *P. aeruginosa* suggests that this reagent could serve as an effective passive therapeutic reagent for most strains of *P. aeruginosa.* Essentially all isolates carry the alginate biosynthetic genes, and most clinical non-mucoid isolates produce detectable amounts of alginate in vitro. Interestingly, the alginate-deleted strains of PAO1 and PA 14 were not particularly reduced for virulence in the acute pneumonia model, raising the possibility that alginate-negative variant strains may retain significant virulence potential in this setting. However, alginate was shown to be an essential factor for infecting transgenic CF mice with either non-mucoid or mucoid strains (47) suggesting that in the setting of CF, alginate-negative variants would be poorly virulent. Indeed, in this clinical setting the conversion of non-mucoid to mucoid strains signals the onset of increased deterioration in lung function (5, 6) which may indicate that initiation of therapy with a mAb such as F429 when the first non-mucoid isolates *of P. aeruginosa* are obtained could help control this variant and prevent emergence of the more pathogenic mucoid variety.

### References

1. Pugashetti, B. K., H. M. Metzger, Jr., L. Vadas, and D. S. Feingold. 1982. Phenotypic differences among clinically isolated mucoid Pseudomonas aeruginosa strains. J. Clin. Microbiol. 16:686*.*
2. Foundation, C. F. 2003. Patient Registry 2002. Annual Report, Bethesda, MD, September, 2003.
3. Burns, J. L., R. L. Gibson, S. McNamara, D. Yim, J. Emerson, M. Rosenfeld, P. Hiatt, K. McCoy, R. Castile, A. L. Smith, and B. W. Ramsey. 2001. Longitudinal assessment of Pseudomonas aeruginosa in young children with cystic fibrosis. J. Infect. Dis. 183:444*.*
4. Kosorok, M. R., L. Zeng, S. E. West, M. J. Rock, M. L. Splaingard, A. Laxova, C. G. Green, J. Collins, and P. M. Farrell. 2001. Acceleration of lung disease in children with cystic fibrosis after Pseudomonas aeruginosa acquisition. Pediatr. Pulmonol. 32:277.
5. Pedersen, S. S., N. Hoiby, F. Espersen, and C. Koch. 1992. Role of alginate in infection with mucoid Pseudomonas aeruginosa in cystic fibrosis. Thorax 47:6*.*
6. Demko, C. A., P. J. Byard, and P. B. Davis. 1995. Gender differences in cystic fibrosis: Pseudomonas aeruginosa infection. J. Clin. Epidemiol. 48:1041*.*
7. Pier, G. B., J. M. Saunders, P. Ames, M. S. Edwards, H. Auerbach, J. Goldfarb, D. P. Speert, and S. Hurwitch. 1987. Opsonophagocytic killing antibody to Pseudomonas aeruginosa mucoid exopolysaccharide in older, non-colonized cystic fibrosis patients. N. Engl. J. Med. 317:793*.*
8. Parad, R. B., C. J. Gerard, D. Zurakowski, D. P. Nichols, and G. B. Pier. 1999. Pulmonary outcome in cystic fibrosis is influenced primarily by mucoid Pseudomonas aeruginosa infection and immune status and only modestly by genotype. Infect. Immun. 67:4744*.*
9. Pier, G. B., M. Grout, and D. DesJardins. 1991. Complement deposition by antibodies to Pseudomonas aeruginosa mucoid exopolysaccharide (MEP) and by non-MEP specific opsonins. J. Immunol. 147:1869*.*
10. Ames, P., D. DesJardins, and G. B. Pier. 1985. Opsonophagocytic killing activity of rabbit antibody to Pseudomonas aeruginosa mucoid exopolysaccharide. Infect. Immun. 49:281*.*
11. Garner, C. V., D. DesJardins, and G. B. Pier. 1990. Immunogenic properties of Pseudomonas aeruginosa mucoid exopolysaccharide. Infect. Immun. 58:1835*.*
12. Garner, C. V., and G. Pier, B. 1988. Human immune response to Pseudomonas aeruginosa mucoid exopolysaccharide vaccine. Clin. Res. 36:465A*.*
13. Pier, G. B., D. DesJardins, M. Grout, C. Garner, S. E. Bennett, G. Pekoe, S. A. Fuller, M. O. Thornton, W. S. Harkonen, and H. C. Miller. 1994. Human immune response to Pseudomonas aeruginosa mucoid exopolysaccharide (alginate) vaccine. Infect. Immun. 62:3972*.*
14. Fuller, S., M. Propst, L. Gross, S. Krentz, S. Stewart, M. Thornton, and G. Pier. 1994. An immune globulin specific to Pseudomonas aeruginosa mucoid exopolysaccharide (MEP) promotes opsonic killing of isolates from cystic fibrosis (CF) patients. Abstracts of the 34th Interscience Conference on Antimicrobial Agents and Chemotherapy, Abstract G7, pg. 23.
15. Pier, G. B., F. Coleman, M. Grout, M. Franklin, and D. E. Ohman. 2001. Role of alginate O acetylation in resistance of mucoid Pseudomonas aeruginosa to opsonic phagocytosis. Infect. Immun. 69:1895*.*
16. Meluleni, G. J., M. Grout, D. J. Evans, and G. B. Pier. 1995. Mucoid Pseudomonas aeruginosa growing in a biofilm in vitro are killed by opsonic antibodies to the mucoid exopolysaccharide capsule but not by antibodies produced during chronic lung infection in cystic fibrosis patients. J Immunol. 155:2029*.*
17. Anastassiou, E. D., A. S. Mintzas, C. Kounavis, and G. Dimitracopoulos. 1987. Alginate production by clinical nonmucoid Pseudomonas aeruginosa. J. Clin. Microbiol. 25: 656*.*
18. Pier, G. B., D. DesJardins, T. Aguilar, M. Barnard, and D. P. Speert. 1986. Polysaccharide surface antigens expressed by non-mucoid isolates of Pseudomonas aeruginosa from cystic fibrosis patients. J. Clin. Microbiol. 24:189*.*
19. Allewelt, M., F. T. Coleman, M. Grout, G. P. Priebe, and G. B. Pier. 2000. Acquisition of expression of the Pseudomonas aeruginosa ExoU cytotoxin leads to increased bacterial virulence in a murine model of acute pneumonia and systemic spread. Infect. Immun. 68:3998*.*
20. Theilacker, C., F. Coleman, S. Mueschenborn, M. Grout, and G. B. Pier. 2003. Construction and characterization of a Pseudomonas aeruginosa mucoid exopolysaccharide/alginate conjugate vaccine. Infect. Immun. 71:3875*.*
21. Chitnis, C. E., and D. E. Ohman. 1990. Cloning of Pseudomonas aeruginosa algG, which controls alginate structure. J. Bacteriol. 172:2894*.*
22. Penaloza-Vazquez, A., S. P. Kidambi, A. M. Chakrabarty, and C. L. Bender. 1997. Characterization of the alginate biosynthetic gene cluster in Pseudomonas syringae pv. syringae. J Bacteriol 179:4464*.*
23. Hermanson, G. T. 1996. Bioconjugate Techniques. Academic Press, San Diego, CA.
24. Silo-Suh, L., S. J. Suh, P. A. Sokol, and D. E. Ohman. 2002. A simple alfalfa seedling infection model for Pseudomonas aeruginosa strains associated with cystic fibrosis shows AlgT (sigma-22) and RhlR contribute to pathogenesis. Proc Natl Acad Sci US A 99:15699*.*
25. Yorgey, P., L. G. Rahme, M. W. Tan, and F. M. Ausubel. 2001. The roles ofmucD and alginate in the virulence of Pseudomonas aeruginosa in plants, nematodes and mice. Mol. Microbiol. 41:1063*.*
26. Posner, M. R., H. S. Elboim, and M. B. Tumber. 1990. Epstein Barr virus transformation of peripheral blood B cells secreting antibodies reactive with cell surface antigens. Autoimmunity 8:149*.*
27. Posner, M. R., H. Elboim, and D. Santos. 1987. The construction and use of a human-mouse myeloma analogue suitable for the routine production of hybridomas secreting human monoclonal antibodies. Hybridoma 6:611*.*
28. Preston, M. J., A. A. Gerceker, M. E. Reff, and G. B. Pier. 1998. Production and characterization of a set of mouse-human chimeric immunoglobulin G (IgG) subclass and IgA monoclonal antibodies with identical variable regions specific for Pseudomonas aeruginosa serogroup 06 lipopolysaccharide. Infect. Immun. 66:4137*.*
29. Reff, M. E., K. Carner, K. S. Chambers, P. C. Chinn, J. E. Leonard, R. Raab, R. A. Newman, N. Hanna, and D. R. Anderson. 1994. Depletion of B cells in vivo by a chimeric mouse human antibody to CD20. Blood 83:435*.*
30. Priebe, G. P., G. J. Meluleni, F. T. Coleman, J. B. Goldberg, and G. B. Pier. 2003. Protection against fatal Pseudomonas aeruginosa pneumonia in mice after nasal immunization with a live, attenuated aroA deletion mutant. Infect. Immun. 71:1453*.*
31. Wozniak, D. J., T. J. Wyckoff, M. Starkey, R. Keyser, P. Azadi, G. A. O'Toole, and M. R. Parsek. 2003. Alginate is not a significant component of the extracellular polysaccharide matrix of PA14 and PAO1 Pseudomonas aeruginosa biofilms. Proc. Natl. Acad. Sci. U. S. A. 100:7907*.*
32. Roy-Burman, A., R. H. Savel, S. Racine, B. L. Swanson, N. S. Revadigar, J. Fujimoto, T. Sawa, D. W. Frank, and J. P. Wiener-Kronish. 2001. Type III protein secretion is associated with death in lower respiratory and systemic Pseudomonas aeruginosa infections. J. Infect. Dis. 183:1767*.*
33. Hauser, A. R., E. Cobb, M. Bodi, D. Mariscal, J. Valles, J. N. Engel, and J. Rello. 2002. Type III protein secretion is associated with poor clinical outcomes in patients with ventilator-associated pneumonia caused by Pseudomonas aeruginosa. Crit. Care Med. 30:521*.*
34. Doring, G., and F. Dorner. 1997. A multicenter vaccine trial using the Pseudomonas aeruginosa flagella vaccine IMMUNO in patients with cystic fibrosis. Behring. Inst. Mitt.:338*.*
35. Ratjen, F., and G. Doring. 2003. Cystic fibrosis. Lancet 361:681*.*
36. Lai, H. P., P. R. Hsueh, Y. C. Chen, P. I. Lee, C. Y. Lu, M. Y. Lu, W. C. Lin, Y. C. Hsieh, C. Y. Lee, K. H. Lin, and L. M. Huang. 2003. Bacteremia in hematological and oncological children with febrile neutropenia: experience in a tertiary medical center in Taiwan. J Microbiol Immunol Infect 36:197*.*
37. Hamzeh, F., S. S. Kanj, and M. Uwaydah. 2000. Febrile neutropenia in cancer patients in a tertiary care medical center in Lebanon: microbial spectrum and outcome. J Med Liban 48:136*.*
38. Pruitt, B. A., Jr., A. T. McManus, S. H. Kim, and C. W. Goodwin. 1998. Bum wound infections: current status. World J. Surg. 22:135*.*
39. Ewig, S., A. Torres, M. El-Ebiary, N. Fabregas, C. Hernandez, J. Gonzalez, J. M. Nicolas, and L. Soto. 1999. Bacterial colonization patterns in mechanically ventilated patients with traumatic and medical head injury. Incidence, risk factors, and association with ventilator-associated pneumonia. Am. J. Respir. Crit. Care Med 159:188*.*
40. Fenton, C., L. J. Scott, and G. L. Plosker. 2004. Palivizumab: a review of its use as prophylaxis for serious respiratory syncytial virus infection. Paediatr Drugs 6:177*.*
41. Jones, R. J., E. A. Roe, and J. L. Gupta. 1980. Controlled trial of Pseudomonas immunoglobulin and vaccine in burn patients. Lancet 2:1263*.*
42. Roe, E. A., and R. J. Jones. 1983. Immunization of burned patients against Pseudomonas aeruginosa infection at Safdarjang Hospital, New Delhi. Rev. Infect. Dis. 5:S922*.*
43. Cryz Jr., S. J., E. Furer, J. C. Sadoff, T. Fredeking, J. U. Que, and A. S. Cross. 1991. Production and characterization of a human hyperimmune intravenous immunoglobulin against Pseudomonas aeruginosa and Klebsiella species. J. Infect. Dis 163:1055*.*
44. Cryz Jr., S. J., J. C. Sadoff, and E. Fürer. 1989. Octavalent Pseudomonas aeruginosa O-polysaccharide-toxin A conjugate vaccine. Microb. Pathog. 6:75*.*
45. Donta, S. T., P. Peduzzi, A. S. Cross, J. Sadoff, C. Haakenson, S. J. Cryz, C. Kauffman, S. Bradley, G. Gafford, D. Elliston, T. R. Beam, J. F. John, B. Ribner, R. Cantey, C. H. Welsh, R. T. Ellison, E. J. Young, R. J. Hamill, H. Leaf, R M. H. Schein, M. Mulligan, C. Johnson, E. Abrutyn, J. M. Griffiss, R Hamadeh, A. H. Eliasson, J. B. Mcclain, G. P. Melcher, J. W. Kelly, W. R. Byrne, M. Wallace, D. Amundson, B. Gumpert, and D. Slagle. 1996. Immunoprophylaxis against Klebsiells and Pseudomonas aeruginosa infections. J. Infect. Dis. 174:537*.*
46. Pier, G. B., G. J. Small, and H. B. Warren. 1990. Protection against mucoid Pseudomonas aeruginosa in rodent models of endobronchial infection. Science 249:537*.*
47. Coleman, F. T., S. Mueschenborn, G. Meluleni, C. Ray, V. J. Carey, S. O. Vargas, C. L. Cannon, F. M. Ausubel, and G. B. Pier. 2003. Hypersusceptibility of cystic fibrosis mice to chronic Pseudomonas aeruginosa oropharyngeal colonization and lung infection. Proc. Natl. Acad Sci. U S A 100:1949*.*

## Claims

1. A method for identifying mannuronic acid binding peptides in vitro, the method comprising:
contacting a candidate peptide to isolated beta-1,4 linked homopolymer of D-mannuronic acid in vitro, and
determining whether the candidate peptide binds to an epitope that comprises an intact carboxyl group on C6 of the isolated beta-1,4 linked homopolymer of D-mannuronic acid.

2. The method of claim 1, wherein determining whether the candidate peptide binds to the epitope comprises comparing the level of binding of the candidate peptide to the epitope with a level of binding of a control to the epitope.

3. The method of claim 2, wherein the control is a negative control level of binding and the candidate peptide is a mannuronic acid binding peptide if the test level of binding is greater than the negative control level of binding, in particular wherein the test level of binding is greater than the negative control level of binding by at least 2-fold, at least 3-fold, at least 4-fold, at least 5-fold, at least 10-fold, at least 20-fold, at least 50-fold or at least 100-fold.

4. The method of claim 2, wherein the control is a positive control level of binding and the candidate peptide is a mannuronic acid binding peptide if the test level of binding is substantially equal to or greater than the positive control level of binding, in particular wherein the test level of binding is within +/- 50%, within +/- 25%, or within +/- 10% of the positive control level of binding.

5. The method of claim 1, wherein the beta-1,4 linked homopolymer of D-mannuronic acid and candidate peptide are both soluble, or wherein the beta-1,4 linked homopolymer of D-mannuronic acid and the candidate peptide are both labeled, in particular wherein the beta-1,4 linked homopolymer of D-mannuronic acid and the candidate peptide are labeled with a FRET pair.

6. The method of claim 1, further comprising excluding candidate peptides that bind to mannoside, or further comprising excluding candidate peptides that bind to guluronic acid.

7. A method for identifying mannuronic acid binding peptides in vitro, the method comprising:
determining the level of binding of monoclonal antibody F428 or F429 or an antigen-binding fragment thereof to isolated beta-1,4 linked homopolymer of D-mannuronic acid in the presence and absence of a candidate peptide in vitro,
wherein a candidate peptide that reduces the level of binding of monoclonal antibody F428 or F429 or an antigen-binding fragment thereof to isolated beta-1,4 linked homopolymer of D-mannuronic acid, and does not bind to the monoclonal antibody, or antigen-binding fragment thereof is a mannuronic acid binding peptide.

8. The method of claim 7, wherein the candidate peptide reduces the level of binding of monoclonal antibody F428 or F429 or an antigen-binding fragment thereof to isolated beta-1,4 linked homopolymer of D-mannuronic acid by at least 2-fold, at least 3-fold, at least 4-fold, at least 5-fold, at least 10-fold, at least 20-fold, at least 50-fold or at least 100-fold.

9. The method of claim 1 or 7, wherein the candidate peptide is an antibody or a fragment thereof, in particular a single chain antibody, or wherein the candidate peptide is a library member, in particular a synthetic library member, or a naturally occurring library member.

10. The method of claim 1 or 7, wherein the beta-1,4 linked homopolymer of D-mannuronic acid is an oligomer, in particular wherein the oligomer has a length of two monomers, four monomers, six monomers, ten monomers, fifteen monomers or twenty monomers or wherein the beta-1,4 linked homopolymer of D-mannuronic acid is a polymer, more in particular wherein the polymer has a length of at least 21, at least 50, at least 100, at least 500 monomers, at least 1,000 monomers, at least 5,000 monomers, at least 10,000 monomers, or at least 100,000 monomers.

11. The method of claim 1 or 7, wherein the beta-1,4 linked homopolymer of D-mannuronic acid is acetylated, in particular wherein the beta-1,4 linked homopolymer of D-mannuronic acid is O-acetylated at one or both C2 or C3, or wherein the beta-1,4 linked homopolymer of D-mannuronic acid is not acetylated, or wherein the candidate peptide does not bind to guluronic acid, in particular wherein the beta-1,4 linked homopolymer of D-mannuronic acid is conjugated to at least one guluronic acid monomer.

12. The method of claim 7, wherein the monoclonal antibody or antigen-binding fragment thereof, the beta-1,4 linked homopolymer of D-mannuronic acid and the candidate peptide are soluble.

13. The method of claim 7, wherein the monoclonal antibody or antigen-binding fragment thereof is fixed, or wherein the monoclonal antibody or antigen-binding fragment thereof is labeled.

14. The method of claim 1 or 7, wherein the beta-1,4 linked homopolymer of D-mannuronic acid, or the candidate peptide is fixed.

15. The method of claim 1 or 7, wherein the beta-1,4 linked homopolymer of D-mannuronic acid or the candidate peptide is labeled.

16. The method of claim 7, wherein the monoclonal antibody or antigen-binding fragment thereof and the beta-1,4 linked homopolymer of D-mannuronic acid are both labeled, in particular wherein the monoclonal antibody or antigen-binding fragment thereof and the beta-1,4 linked homopolymer of D-mannuronic acid are labeled with a FRET pair.

17. The method of claim 1 or 7, wherein the candidate peptide is not positively charged.

18. The method of claim 1 or 7, further comprising excluding candidate peptides that bind to mannoside, or further comprising excluding candidate peptides that bind to guluronic acid.

19. A composition comprising a peptide that binds specifically to an epitope comprising a carboxyl group on C6 of a mannuronic acid, wherein the peptide is none of monoclonal antibody F428, F429, or a monoclonal antibody comprising the amino acid sequences of all complementarity determining regions of F428 or F429.

20. The composition of claim 19, wherein the peptide is a monoclonal antibody, in particular wherein the monoclonal antibody is a human or humanized antibody, or wherein the peptide is an antibody fragment or a single chain antibody.

21. The composition of claim 19, wherein the composition is a pharmaceutical preparation.

22. The composition of claim 19, wherein the peptide is isolated.

23. An antibody or fragment thereof that binds to an epitope that comprises an intact carboxyl group on C6 of a mannuronic acid for use in a method of inducing passive immunity in a subject, wherein the antibody or fragment thereof that binds to an epitope that comprises an intact carboxyl group on C6 of a mannuronic acid is administered to a subject in need thereof in an effective amount to induce passive immunity, wherein the antibody or antibody fragment is not monoclonal antibody F428 or F429 or an antigen-binding fragment thereof, and is not a polyclonal antibody.

24. The antibody or fragment thereof of claim 23, wherein the passive immunity is against bacteria.

25. The antibody or fragment thereof of claim 23, wherein the bacteria is a *Pseudomonas* spp, in particular wherein the *Pseudomonas* spp. is *P. aeruginosa, P. fluorescens, P. syringae, P. putida* or *P. mendocina,* or wherein the *Pseudomonas* spp. is a mucoid strain or a non-mucoid strain, or in particular wherein the bacteria is an *Azotobacter* spp, more in particular wherein the *Azotobacter* spp. is *A. vinelandii.*

26. The antibody or fragment thereof of claim 23, wherein the antibody or fragment thereof is a monoclonal antibody, in particular wherein the monoclonal antibody is a human or humanized antibody, or wherein the antibody or fragment thereof is an antibody fragment or a single chain antibody.

27. The antibody or fragment thereof of claim 23, wherein the subject has or is at risk of a *Pseudomonas* infection, in particular wherein the *Pseudomonas* infection is *P. aeruginosa* infection, *P. syringae* infection, *P. fluorescens* infection, *P. putida* infection or *P. mendocina* infection, or wherein the subject or is at risk of an *Azotobacter* infection, in particular wherein the *Azotobacter* infection is *A. vinelandii* infection, or wherein the subject has a respiratory condition, in particular wherein the subject is receiving mechanical ventilation, more in particular wherein the subject has ventilation-associated pneumonia, or wherein the subject has cystic fibrosis or cancer, or wherein the subject has been burned, or wherein the subject is a human.

28. The antibody or fragment thereof of claim 23, wherein the antibody or fragment thereof is isolated.

## Patentansprüche

1. Verfahren zur in vitro Identifikation von Mannuronsäure-bindenden Peptiden, das Verfahren umfassend:
In vitro in Kontakt bringen eines Kandidatpeptids mit isoliertem beta-1,4 verbundenem Homopolymer von D-Mannuronsäure, und
Bestimmen, ob das Kandidatpeptid an ein Epitop bindet, das eine intakte Carboxylgruppe am C6 des isolierten beta-1,4 verbundenen Homopolymers von D-Mannuronsäure umfasst.

2. Verfahren nach Anspruch 1, wobei das Bestimmen, ob das Kandidatpeptid an ein Epitop bindet, das Vergleichen des Bindungsgrads des Kandidatpeptids an ein Epitop mit einem Bindungsgrad einer Kontrolle an das Epitop umfasst.

3. Verfahren nach Anspruch 2, wobei die Kontrolle eine Negativ-Bindungsgradkontrolle ist und das Kandidatpeptid ein Mannuronsäure-bindendes Peptid ist, wenn der Testgrad an Bindung größer als die Negativ-Bindungsgradkontrolle ist, insbesondere wobei der Testgrad an Bindung größer als die Negativ-Bindungsgradkontrolle um das mindestens 2-fache, mindestens 3-fache, mindestens 4-fache, mindestens 5-fache, mindestens 10-fache, mindestens 20-fache, mindestens 50-fache oder mindestens 100-fache ist.

4. Verfahren nach Anspruch 2, wobei die Kontrolle eine Positiv-Bindungsgradkontrolle ist und das Kandidatpeptid ein Mannuronsäure-bindendes Peptid ist, wenn der Testgrad an Bindung im Wesentlichen gleich oder größer als die Positiv-Bindungsgradkontrolle ist, insbesondere wobei der Testgrad an Bindung innerhalb von +/- 50%, innerhalb von +/- 25%, oder innerhalb von +/- 10% der Positiv-Bindungsgradkontrolle ist.

5. Verfahren nach Anspruch 1, wobei das beta-1,4 verbundene Homopolymer von D-Mannuronsäure und das Kandidatpeptid beide löslich sind, oder wobei das beta-1,4 verbundene Homopolymer von D-Mannuronsäure und das Kandidatpeptid beide markiert sind, insbesondere wobei das beta-1,4 verbundene Homopolymer von D-Mannuronsäure und das Kandidatpeptid mit einem FRET-Paar markiert ist.

6. Verfahren nach Anspruch 1, ferner umfassend das Ausschließen von Kandidatpeptiden, die an Mannosid binden, oder ferner umfassend das Ausschließen von Kandidatpeptiden, die an Guluronsäure binden.

7. Verfahren zur in vitro Identifikation von Mannuronsäure-bindenden Peptiden, das Verfahren umfassend:
Bestimmen des Bindungsgrads an monoklonalem Antikörper F428 oder F429 oder ein Antigen-bindendes Fragment davon an isoliertem beta-1,4 verbundenem Homopolymer von D-Mannuronsäure in der Gegenwart und
Abwesenheit eine Kandidatpeptipds in vitro,
wobei ein Kandidatpeptid, dass den Bindungsgrad an monoklonalem Antikörper F428 oder F429 oder ein Antigen-bindendes Fragment davon an isoliertem beta-1,4 verbundenem Homopolymer von D-Mannuronsäure verringert und nicht an den monoklonalen Antikörper, oder an ein Antigen-bindendes Fragment davon bindet, ein Mannuronsäure-bindedes Peptid ist.

8. Verfahren nach Anspruch 7, wobei das Kandidatpeptid den Bindungsgrad an monoklonalem Antikörper F428 oder F429 oder einem Antigen-bindenden Fragment davon an isoliertem beta-1,4 verbundenem Homopolymer von D-Mannuronsäure um das mindestens 2-fache, mindestens 3-fache, mindestens 4-fache, mindestens 5-fache, mindestens 10-fache, mindestens 20-fache, mindestens 50-fache oder mindestens 100-fache verringert.

9. Verfahren nach Anspruch 1 oder 7, wobei das Kandidatpeptid ein Antikörper oder ein Fragment davon, insbesondere ein Einzelketten-Antikörper ist, oder wobei das Kandidatpeptid ein Bibliotheksmitglied ist, insbesondere ein synthetisches Bibliotheksmitglied, oder ein natürlich auftretendes Bibliotheksmitglied.

10. Verfahren nach Anspruch 1 oder 7, wobei das beta-1,4 verbundene Homopolymer von D-Mannuronsäure ein Oligomer ist, insbesondere wobei das Oligomer eine Länge von zwei Monomeren, vier Monomeren, sechs Monomeren, zehn Monomeren, fünfzehn Monomeren oder zwanzig Monomeren hat oder wobei das beta-1,4 verbundene Homopolymer von D-Mannuronsäure ein Polymer ist, vor allem insbesondere wobei das Polymer eine Länge von mindestens 21, mindestens 50, mindestens 100, mindestens 500 Monomeren, mindestens 1.000 Monomeren, mindestens 5.000 Monomeren, mindestens 10.000 Monomeren oder mindestens 100.000 Monomeren hat.

11. Verfahren nach Anspruch 1 oder 7, wobei das beta-1,4 verbundene Homopolymer von D-Mannuronsäure acetyliert ist, insbesondere wobei das beta-1,4 verbundene Homopolymer von D-Mannuronsäure O-acetyliert an einem oder beiden der C2 oder C3 ist, oder wobei das beta-1,4 verbundene Homopolymer von D-Mannuronsäure nicht acetyliert ist, oder wobei das Kandidatpeptid nicht an Guluronsäure bindet, insbesondere wobei das beta-1,4 verbundene Homopolymer von D-Mannuronsäure an mindestens ein Guluronsäure-Monomer konjugiert ist.

12. Verfahren nach Anspruch 7, wobei der monoklonale Antikörper oder ein Antigen-bindendes Fragment davon, das beta-1,4 verbundene Homopolymer von D-Mannuronsäure und das Kandidatpeptid löslich sind.

13. Verfahren nach Anspruch 7, wobei der monoklonale Antikörper oder ein Antigen-bindendes Fragment davon fixiert ist, oder wobei der monoklonale Antikörper oder ein Antigen-bindendes Fragment davon markiert ist.

14. Verfahren nach Anspruch 1 oder 7, wobei das beta-1,4 verbundene Homopolymer von D-Mannuronsäure oder das Kandidatpeptid fixiert ist.

15. Verfahren nach Anspruch 1 oder 7, wobei das beta-1,4 verbundene Homopolymer von D-Mannuronsäure oder das Kandidatpeptid markiert ist.

16. Verfahren nach Anspruch 7, wobei der monoklonale Antikörper oder ein Antigen-bindendes Fragment davon und das beta-1,4 verbundene Homopolymer von D-Mannuronsäure beide markiert sind, insbesondere wobei der monoklonale Antikörper oder ein Antigen-bindendes Fragment davon und das beta-1,4 verbundene Homopolymer von D-Mannuronsäure mit einem FRET-Paar markiert sind.

17. Verfahren nach Anspruch 1 oder 7, wobei das Kandidatpeptid nicht positiv geladen ist.

18. Verfahren nach Anspruch 1 oder 7, ferner umfassend das Ausschließen von Kandidatpeptiden, die an Mannosid binden, oder ferner umfassend das Ausschließen von Kandidatpeptiden, die an Guluronsäure binden.

19. Zusammensetzung umfassend ein Peptid, das spezifisch an ein Epitop bindet, umfassend eine Carboxylgruppe am C6 einer Mannuronsäure, wobei das Peptid kein monoklonaler Antikörper F428, F429, oder ein monoklonaler Antikörper umfassend die Aminosäuresequenzen aller Komplementaritätsregionen (complementarity determining regions) von F428 oder F429 umfasst.

20. Zusammensetzung nach Anspruch 19, wobei das Peptid ein monoklonaler Antikörper ist, insbesondere wobei der monoklonale Antikörper ein humaner oder humanisierter Antikörper ist, oder wobei das Peptid ein Antikörper-Fragment oder ein Einzelketten-Antikörper ist.

21. Zusammensetzung nach Anspruch 19, wobei die Zusammensetzung ein pharmazeutisches Präparat ist.

22. Zusammensetzung nach Anspruch 19, wobei das Peptid isoliert ist.

23. Antikörper oder Fragment davon, das an ein Epitop bindet, das eine intakte Carboxylgruppe am C6 einer Mannuronsäure umfasst, zur Verwendung in einem Verfahren zur Induzierung passiver Immunität in einem Subjekt, wobei der Antikörper oder das Fragment davon, der oder das an ein Epitop bindet, der eine intakte Carboxylgruppe am C6 von Mannuronsäure umfasst an ein Subjekt, das den. Bedarf hat, in einer effektiven Menge verabreicht wird, um passive Immunität zu induzieren, wobei der Antikörper oder das Antikörper-Fragment nicht monoklonaler Antikörper F428 oder F429 oder ein Antigen-bindendes Fragment davon ist und nicht ein polyklonaler Antikörper ist.

24. Antikörper oder Fragment davon nach Anspruch 23, wobei die passive Immunität gegen Bakterien ist.

25. Antikörper oder Fragment davon nach Anspruch 23, wobei das Bakterium ein *Pseudomonas spp,* insbesondere wobei der *Pseudomonas spp. P. aeruginosa* ist, *P. fluorescens, P. syringae, P. putida* oder *P. mendocina,* oder wobei der *Pseudomonas spp.* ein Mucoid-Stamm oder ein nicht-Mucoid-Stamm ist, oder insbesondere wobei das Bakterium ein *Azotobacter spp,* vor allem insbesondere wobei das *Azotobacter spp. A. vinelandii* ist.

26. Antikörper oder das Fragment davon nach Anspruch 23, wobei der Antikörper oder das Fragment davon ein monoklonaler Antikörper ist, insbesondere wobei der monoklonale Antikörper ein humaner oder humanisierter Antikörper ist, oder wobei der Antikörper oder das Fragment davon ein Antikörper-Fragment oder ein Einzelketten-Antikörper ist.

27. Antikörper oder das Fragment davon nach Anspruch 23, wobei das Subjekt eine *Pseudomonas* Infektion hat oder das Risiko hierzu hat, insbesondere wobei die *Pseudomonas* Infektion eine *P. aeruginosa* Infektion ist, *P. syringae* Infektion, *P. fluorescens* Infektion, *P. putida* Infektion oder *P. mendocina* Infektion ist, oder wobei das Subjekt eine *Azotobacter* Infektion hat oder das Risiko dazu hat, insbesondere wobei die *Azotobacter* Infektion eine *A. vinelandii* Infektion ist, oder wobei das Subjekt einen respiratorischen Zustand hat, insbesondere wobei das Subjekt künstliche Beatmung erhält, vor allem insbesondere wobei das Subjekt eine Beatmungsassoziierte-Pneumonie hat, oder wobei das Subjekt zystische Fibrose oder Krebs hat, oder wobei das Subjekt Verbrennungen hat, oder wobei das Subjekt ein Mensch ist.

28. Antikörper oder Fragment davon nach Anspruch 23, wobei der Antikörper oder das Fragment davon isoliert ist.

## Revendications

1. Procédé d'identification de peptides se liant à l'acide mannuronique in vitro, le procédé comprenant :
la mise en contact d'un peptide candidat avec un homopolymère d'acide D-mannuronique lié en bêta-1,4 isolé in vitro, et
la détermination du fait que le peptide candidat se lie à un épitope qui comprend un groupe carboxyle intact en C6 de l'homopolymère d'acide D-mannuronique lié en bêta-1,4 isolé.

2. Procédé selon la revendication 1, dans lequel la détermination du fait que le peptide candidat se lie à l'épitope comprend la comparaison du niveau de liaison du peptide candidat à l'épitope avec le niveau de liaison d'un témoin à l'épitope.

3. Procédé selon la revendication 2, dans lequel le témoin est un niveau de liaison d'un témoin négatif et le peptide candidat est un peptide se liant à l'acide mannuronique si le niveau de liaison du test est supérieur au niveau de liaison du témoin négatif, en particulier dans lequel le niveau de liaison du test est supérieur au niveau de liaison du témoin négatif dans un rapport d'au moins 2, d'au moins 3, d'au moins 4, d'au moins 5, d'au moins 10, d'au moins 20, d'au moins 50 ou d'au moins 100.

4. Procédé selon la revendication 2, dans lequel le témoin est un niveau de liaison d'un témoin positif et le peptide candidat est un peptide se liant à l'acide mannuronique si le niveau de liaison du test est substantiellement supérieur ou égal au niveau de liaison du témoin positif, en particulier dans lequel le niveau de liaison du test est égal à ±50 %, à ±25 %, ou à ±10 %, du niveau de liaison du témoin positif.

5. Procédé selon la revendication 1, dans lequel l'homopolymère d'acide D-mannuronique lié en bêta-1,4 et le peptide candidat sont tous les deux solubles, ou dans lequel l'homopolymère d'acide D-mannuronique lié en bêta-1,4 et le peptide candidat sont tous les deux marqués, en particulier dans lequel l'homopolymère d'acide D-mannuronique lié en bêta-1,4 et le peptide candidat sont marqués avec une paire FRET.

6. Procédé selon la revendication 1, comprenant en outre l'exclusion des peptides candidats qui se lient au mannoside, ou comprenant en outre l'exclusion des peptides candidats qui se lient à l'acide guluronique.

7. Procédé d'identification de peptides se liant à l'acide mannuronique in vitro, le procédé comprenant :
la détermination du niveau de liaison d'un anticorps monoclonal F428 ou F429 ou d'un fragment de liaison à l'antigène de celui-ci, à un homopolymère d'acide D-mannuronique lié en bêta-1,4 isolé en présence et en l'absence d'un peptide candidat in vitro,
dans lequel un peptide candidat qui réduit le niveau de liaison de l'anticorps monoclonal F428 ou F429 ou d'un fragment de liaison à l'antigène de celui-ci à un homopolymère d'acide D-mannuronique lié en bêta-1,4, et qui ne se lie pas à l'anticorps monoclonal, ou à un fragment de liaison à l'antigène de celui-ci, est un peptide se liant à l'acide mannuronique.

8. Procédé selon la revendication 7, dans lequel le peptide candidat réduit le niveau de liaison de l'anticorps monoclonal F428 ou F429 ou d'un fragment de liaison à l'antigène de celui-ci à un homopolymère d'acide D-mannuronique lié en bêta-1,4 isolé dans un rapport d'au moins 2, d'au moins 3, d'au moins 4, d'au moins 5, d'au moins 10, d'au moins 20, d'au moins 50 ou d'au moins 100.

9. Procédé selon la revendication 1 ou 7, dans lequel le peptide candidat est un anticorps ou un fragment de celui-ci, en particulier un anticorps à chaîne simple, ou dans lequel le peptide candidat est un élément d'une banque, en particulier un élément d'une banque synthétique, ou un élément d'une banque existant à l'état naturel.

10. Procédé selon la revendication 1 ou 7, dans lequel l'homopolymère d'acide D-mannuronique lié en bêta-1,4 est un oligomère, en particulier dans lequel l'oligomère a une longueur de deux monomères, de quatre monomères, de six monomères, de dix monomères, de quinze monomères ou de vingt monomères, ou dans lequel l'homopolymère d'acide D-mannuronique lié en bêta-1,4 est un polymère, plus particulièrement dans lequel le polymère a une longueur d'au moins 21, d'au moins 50, d'au moins 100, d'au moins 500 monomères, d'au moins 1 000 monomères, d'au moins 5 000 monomères, d'au moins 10 000 monomères ou d'au moins 100 000 monomères.

11. Procédé selon la revendication 1 ou 7, dans lequel l'homopolymère d'acide D-mannuronique lié en bêta-1,4 est acétylé, en particulier dans lequel l'homopolymère d'acide D-mannuronique lié en bêta-1,4 est O-acétylé en C2 ou en C3 ou les deux, ou dans lequel l'homopolymère d'acide D-mannuronique lié en bêta-1,4 n'est pas acétylé, ou dans lequel le peptide candidat ne se lie pas à l'acide guluronique, en particulier dans lequel l'homopolymère d'acide D-mannuronique lié en bêta-1,4 est conjugué à au moins un monomère d'acide guluronique.

12. Procédé selon la revendication 7, dans lequel l'anticorps monoclonal ou le fragment de liaison à l'antigène de celui-ci, l'homopolymère d'acide D-mannuronique lié en bêta-1,4 et le peptide candidat sont solubles.

13. Procédé selon la revendication 7, dans lequel l'anticorps monoclonal ou le fragment de liaison à l'antigène de celui-ci est fixé, ou dans lequel l'anticorps monoclonal ou le fragment de liaison à l'antigène de celui-ci est marqué.

14. Procédé selon la revendication 1 ou 7, dans lequel l'homopolymère d'acide D-mannuronique lié en bêta-1,4 ou le peptide candidat est fixé.

15. Procédé selon la revendication 1 ou 7, dans lequel l'homopolymère d'acide D-mannuronique lié en bêta-1,4 ou le peptide candidat est marqué.

16. Procédé selon la revendication 7, dans lequel l'anticorps monoclonal ou le fragment de liaison à l'antigène de celui-ci et l'homopolymère d'acide D-mannuronique lié en bêta-1,4 sont tous deux marqués, en particulier dans lequel l'anticorps monoclonal ou le fragment de liaison à l'antigène de celui-ci et l'homopolymère d'acide D-mannuronique lié en bêta-1,4 sont marqués avec une paire FRET.

17. Procédé selon la revendication 1 ou 7, dans lequel le peptide candidat n'est pas chargé positivement.

18. Procédé selon la revendication 1 ou 7, comprenant en outre l'exclusion des peptides candidats qui se lient au mannoside, ou comprenant en outre l'exclusion des peptides candidats qui se lient à l'acide guluronique.

19. Composition comprenant un peptide qui se lie spécifiquement à un épitope comprenant un groupe carboxyle en C6 d'un acide mannuronique, dans laquelle le peptide n'est aucun d'un anticorps monoclonal F428, F429 ou d'un anticorps monoclonal comprenant les séquences d'acides aminés de toutes les régions déterminant la complémentarité de F428 ou F429.

20. Composition selon la revendication 19, dans laquelle le peptide est un anticorps monoclonal, en particulier dans laquelle l'anticorps monoclonal est un anticorps humain ou humanisé, ou dans laquelle le peptide est un fragment d'anticorps ou un anticorps à chaîne simple.

21. Composition selon la revendication 19, dans laquelle la composition est une préparation pharmaceutique.

22. Composition selon la revendication 19, dans laquelle le peptide est isolé.

23. Anticorps ou fragment de celui-ci qui se lie à un épitope qui comprend un groupe carboxyle intact en C6 d'un acide mannuronique, pour son utilisation dans un procédé d'induction d'une immunité passive chez un sujet, dans lequel l'anticorps ou le fragment de celui-ci qui se lie à un épitope qui comprend un groupe carboxyle intact en C6 d'un acide mannuronique est administré à un sujet le nécessitant en une quantité efficace pour induire une immunité passive, dans lequel l'anticorps ou le fragment d'anticorps n'est pas un anticorps monoclonal F428 ou F429 ou un fragment de liaison à l'antigène de celui-ci, et n'est pas un anticorps polyclonal.

24. Anticorps ou fragment de celui-ci selon la revendication 23, dans lequel l'immunité passive est dirigée contre une bactérie.

25. Anticorps ou fragment de celui-ci selon la revendication 23, dans lequel la bactérie est une *Pseudomonas* spp, en particulier dans lequel la *Pseudomonas* spp est *P. aeruginosa, P. fluorescens, P. syringae, P. putida* ou *P. mendocina,* ou dans lequel la *Pseudomonas* spp est une souche mucoïde ou une souche non mucoïde, ou en particulier dans lequel la bactérie est une *Azotobacter* spp, plus particulièrement dans lequel *l'Azotobacter* spp est *A. vinelandii.*

26. Anticorps ou fragment de celui-ci selon la revendication 23, dans lequel l'anticorps ou le fragment de celui-ci est un anticorps monoclonal, en particulier dans lequel l'anticorps monoclonal est un anticorps humain ou humanisé, ou dans lequel l'anticorps ou le fragment de celui-ci est un fragment d'anticorps ou un anticorps à chaîne simple.

27. Anticorps ou fragment de celui-ci selon la revendication 23, dans lequel le sujet a ou présente un risque d'une infection par *Pseudomonas,* en particulier dans lequel l'infection par *Pseudomonas* est une infection par *P. aeruginosa,* une infection par *P. syringae,* une infection par *P. fluorescens,* une infection par *P. putida* ou une infection par *P. mendocina,* ou dans lequel le sujet a ou présente un risque d'une infection par *Azotobacter,* en particulier dans lequel l'infection par *Azotobacter* est une infection par *A. vinelandii,* ou dans lequel le sujet a une affection respiratoire, en particulier dans lequel le sujet reçoit une ventilation mécanique, plus particulièrement dans lequel le sujet a une pneumonie associée à une ventilation, ou dans lequel le sujet a une mucoviscidose ou un cancer, ou dans lequel el sujet a été brûlé, ou dans lequel le sujet est un humain.

28. Anticorps ou fragment de celui-ci selon la revendication 23, dans lequel l'anticorps ou le fragment de celui-ci est isolé.
